# EUROPÄISCHE PATENTSCHRIFT

(11) **EP 4 363 505 B1**
(45) Veröffentlichungstag und Bekanntmachung des Hinweises auf die Patenterteilung: **14.05.2025**
(21) Anmeldenummer: 22741705.2
(22) Anmeldetag: 28.06.2022
(51) Int. Cl.: C08L 97/02, A23L 29/262, C08H 8/00

(54) **BALLASTSTOFFPRÄPARAT AUS MACAUBAFRÜCHTEN UND VERFAHREN ZUR HERSTELLUNG**
DIETARY FIBER PREPARATION FROM MACAUBA FRUIT, AND METHOD OF PRODUCING SAME
PRÉPARATION DE FIBRES ALIMENTAIRES À BASE DE FRUIT DU MACAUBA ET PROCÉDÉ DE FABRICATION ASSOCIÉ

(30) Priorität: 30.06.2021 DE 102021116922
(43) Veröffentlichungstag der Anmeldung: 08.05.2024
(73) Patentinhaber: Fraunhofer-Gesellschaft zur Förderung der angewandten Forschung e.V., 80686 München (DE); Instituto de Technologia de Alimentos (ITAL), 2880 Chácaras Compos dos Amarais Campinas - SP (BR); Instituto Agronômico de Campinas - IAC, 13075-630 Campinas - São Paulo (BR)
(72) Erfinder: TOLEDO E SILVA, Sérgio Henrique, 2880 Chácaras Campos dos Amarais (BR); DOER, Gabriele, 85354 Freising (DE); EISNER, Peter, 85354 Freising (DE); MITTERMAIER, Stefanie, 85354 Freising (DE); MURANYI, Isabel, 85354 Freising (DE); APARECIDA FERRARI, Roseli, 2880 Chácaras Campos dos Amarais (BR); MARTINS MOREIRA, Alexandre, 2880 Chácaras Campos dos Amarais (BR); BATAGLIA DA SILVA, Lidiane, 2880 Chácaras Campos dos Amarais (BR); COLOMBO, Carlos, 2880 Chácaras Campos dos Amarais (BR)
(74) Vertreter: Gagel, Roland
(86) Internationale Anmeldenummer: PCT/EP2022/067694
(87) Internationale Veröffentlichungsnummer: WO 2023/275026

(56) Entgegenhaltungen:
- ANDRADE AMANDA CRISTINA ET AL: "Prebiotic potential of pulp and kernel cake from Jerivá (Syagrus romanzoffiana) and Macaúba palm fruits (Acrocomia aculeata)", FOOD RESEARCH INTERNATIONAL, vol. 136, 1 October 2020 (2020-10-01), AMSTERDAM, NL, pages 109595, XP055968877, ISSN: 0963-9969, DOI: 10.1016/j.foodres.2020.109595
- MICHELLE CARDOSO COIMBRA ET AL: "Proximate composition of guariroba, jerivá and macaúba palm fruits", FOOD RESEARCH INTERNATIONAL, ELSEVIER, AMSTERDAM, NL, vol. 44, no. 7, 12 March 2011 (2011-03-12), pages 2139 - 2142, XP028098973, ISSN: 0963-9969, [retrieved on 20110321], DOI: 10.1016/J.FOODRES.2011.03.032

## Beschreibung

### Anwendungsgebiet

Die Erfindung betrifft funktionelle Ballaststoffpräparate aus Macaubafrüchten, insbesondere für Lebensmittel, Kosmetika, Heimtiernahrung und technische Applikationen, sowie ein Verfahren zur Herstellung dieser Präparate.

### Stand der Technik

Ballaststoffe bestehen hauptsächlich aus Polysacchariden der Pflanzenzellwand (PCW) und Lignin. Pektine, Hemizellulose und Zellulose sind Schlüsselkomponenten der Pflanzenzellwand und liegen je nach Pflanzenart in unterschiedlichen Anteilen vor. Sie umfassen verschiedene Polysaccharidgruppen wie Galacturonane, Arabinane, Xylane, Mannane, Xyloglucane und β-Glucane [1-3]. Die strukturelle Vielfalt, die sich aus der Monosaccharid-Zusammensetzung, den Bindungstypen und - mustern, der Kettenform und dem Polymerisations- und Substitutionsgrad zusammensetzt, legt die spezifischen Eigenschaften von Zellwandpolysacchariden fest.

Natürliche Ballaststoffe und Verdickungsmittel gewinnen für die Human- und Heimtierernährung und für den Einsatz in technischen Applikationen zunehmend an Bedeutung. Die steigende Nachfrage nach ernährungsphysiologisch wirksamen Ballaststoffen, Verdickungsmitteln, technischen Fasern und Rohstoffen für biobasierte Polymere und Verpackungsmaterialen führt zu einem zunehmenden Bedarf an Ballaststoffpräparaten, die einfach und kostengünstig bereitgestellt werden können, die nicht chemisch verändert sind und die in der Herstellung keinen hohen Ressourcenverbrauch verursachen. Mit Ausnahme von einigen wenigen Ballaststoffpräparaten aus Getreide oder Leguminosen, zeigen viele natürliche Ballaststoffe keine guten funktionellen Eigenschaften für Lebensmittel oder für den Einsatz in technischen Applikationen.

Funktionelle Ballaststoffpräparate nach Stand der Technik mit verdickenden, emulgierenden, Schaum- und Gel-bildenden Eigenschaften in wässriger Lösung oder Suspension können zum Beispiel aus Holz, Algen oder aus Mikroorganismen gewonnen werden. Beispiele für derartige hochwertige Präparate sind unter anderem Carboxymethylzellulose, Carrageen, Alginat oder Xanthan. Diese zeigen je nach Rohstoff, Konzentration, pH-Wert und Temperatur spezifische rheologische Eigenschaften in Mischungen mit Wasser, bilden nach dem Erhitzen Gele aus oder können als Filme in Form von Folien oder als Spritzgussteile ausgeformt und so z.B. für Anwendungen als Substitut für erdölbasierte Kunststoffe genutzt werden. Diese Ballaststoffpräparate müssen aber aufwändig aufbereitet und/oder modifiziert werden, so dass hohe Kosten in der Herstellung entstehen und sie aufgrund der umfangreichen Verarbeitungsschritte auch viele Ressourcen verbrauchen.

Hier eröffnet sich ein hohes Potenzial für Verarbeitungsrückstände, die zum Beispiel bei der Gewinnung von Pflanzenöl anfallen. Diese Fraktionen aus Ölpflanzen werden bislang überwiegend als Düngemittel oder Tierfutter genutzt. Sie zeigen aufgrund von antinutritiven Begleitstoffen meist nur eine geringe Wertigkeit für die Tierernährung. Rückstände aus Saaten wie Raps, Sonnenblume oder Lein enthalten nach der Abtrennung des Öls neben Ballaststoff noch Anteile an Schalen und sekundären Pflanzenstoffen. Die daraus resultierende dunkle Farbe, der unangenehm bitter adstringierende oder grasige Geschmack und die z.T. hohe Menge an antinutritiven Komponenten wie Phenolsäuren, cyanogenen Glycosiden, Glucosinulaten, Oligosacchariden und anderen Stoffen macht die meisten Rückstände aus der Pflanzenölgewinnung für den Lebensmittelbereich, für Heimtiernahrung und aufgrund der Farbe auch für viele technische Applikation unbrauchbar.

In diesem Zusammenhang können auch Rückstände aus der Ölgewinnung aus Macaubafrüchten (Acrocomia ssp.) als mögliche Quelle für Ballaststoffpräparate, wie in dieser Erfindung beschrieben, zum Einsatz kommen.

Macauba ist eine Palmenart, die im tropischen und subtropischen Amerika beheimatet ist. Die Früchte der Pflanze bestehen aus einer äußeren Schale (Epikarp), einem öl- und faserreichen Fruchtfleisch (Pulpe), einer inneren Schale (Endokarp) und einem Kern. Das Öl aus der Pulpe wird durch mechanisches Pressen sowie Lösungsmittelextraktion gewonnen [4, 5] und wird hauptsächlich für die Biokraftstoffproduktion verwendet, wobei der entölte faserreiche Zellstoff zurückbleibt. Dieses Nebenprodukt, das etwa 25 % des Gesamtfruchtgewichts ausmacht, wird derzeit als Tierfutter verwendet oder entsorgt [7].

Trotz der bislang kaum erfolgten Nutzung der Pulpe wurde das Potenzial für Lebensmittelanwendungen bereits beschrieben. Vollfettes Macauba-Pulpenmehl wurde in Lebensmitteln wie Keksen, Cupcakes, probiotischen Getränken, Müsliriegeln, Kuchen und Eiscreme verwendet. Der hohe Ölgehalt des Pulpenmehls beeinträchtigt jedoch eine Verwendung als Zutat für die Lebensmittelindustrie vor allem durch Öloxidation und Entwicklung von Oxidationsprodukten mit unangenehmen sensorischen Eigenschaften. Die vorliegende Erfindung zielt hingegen darauf ab, Ballaststoffpräparte mit hoher Funktionalität und guten sensorischen Eigenschaften zu entwickeln. Ob dies möglich ist, war bislang nicht bekannt.

Über die Eignung von Macaubapulpe für technische und biotechnologische Anwendungen ist nach Stand der Technik nur wenig bekannt. Biologisch abbaubare Filme, die mit entöltem Macaubapulpenmehl hergestellt wurden, wurden von da Silva et al. [6] hergestellt. In dieser Arbeit wurde das entfettete Mehl in Wasser dispergiert, mit Glycerin als Weichmacher versetzt und zur Herstellung der Biofilme in Petrischalen gegossen. Die erhaltenen Biofilme waren gelblich gefärbt und opak, was für Folien allerdings unerwünschte Eigenschaften sind.

Im Stand der Technik ist auch die Verwendung von Press- und Lösemittelextraktionsmethoden für die Verarbeitung von Macaubapulpe beschrieben. Pressverfahren umfassen kontinuierliches Pressen oder diskontinuierliches hydraulisches Pressen, während verschiedene Lösemittel wie Hexan, Ethylacetat, Aceton, Methanol, Ethanol und Isopropanol verwendet werden können [4, 5, 6]. Aus diesen Veröffentlichungen geht jedoch nicht hervor, wie sich die spezifische Behandlung auf die Zusammensetzung und die funktionellen Eigenschaften der Rückstände auswirkt. So wird in Trentini et al. [5] die Behandlung von Macaubapulpe mit verschiedenen Lösungsmitteln (Ethylacetat, n-Hexan und Isopropanol) beschrieben. Nach Aussage der Autoren wird weder die Zusammensetzung noch die Funktionalität der entölten Pulpe durch die unterschiedlichen verwendeten Lösungsmittel beeinflusst. Darüber hinaus wird der Einfluss solcher Prozesse auf Schlüsselkomponenten zur Verbesserung der funktionellen und sensorischen Eigenschaften der Ölextraktionsrückstände nicht beschrieben. Ebenfalls unbekannt sind Nachbehandlungen der Rückstände mit dem Ziel, hochfunktioneller Inhaltsstoffe für Lebensmittel, Tiernahrung, Kosmetik und technische Anwendungen zu gewinnen. Die Bereitstellung funktioneller und sensorisch ansprechender Präparate und Zutaten aus Macaubapulpe ist nach dem Stand der Technik somit nicht bekannt.

Auch die Patentliteratur gibt keine Hinweise auf derartige funktionelle und sensorisch vorteilhafte Präparate. So wird in der Schrift BR102012029493-1A2 die Verwendung von vollfetter oder teilweise entfetteter Macaubapulpe für die Nutzung als Tierfutter beschrieben. Diese Schrift beschreibt weder die Verwendung von Macaubapulpe als eine funktionelle Ballaststoffzubereitung noch mögliche Verfahren zu deren Gewinnung. Die BR102014024972-9A2 beschreibt die Verwendung von Macaubapulpenöl als anionischer Kollektor für die Bergbauindustrie. In der BR102016002700-4A2 wird die Verwendung von Macaubapulpe-Presskuchen zur Herstellung von Xylit dargelegt. Dies wird erreicht, indem zuerst die Ballaststoffe aus Macaubapulpe hydrolysiert werden. Ein Hinweis auf die Nutzung der Pulpe für sensorisch ansprechende funktionelle Ballaststoffpräparate wird nicht offenbart. Die PI 0906455-9A2 beschreibt die Verwendung von Macaubapulpe-Presskuchen zur Herstellung von Lipase durch Festphasenfermentation. In der BR102015011035-9A2 wird die Verwendung von Macaubapulpe für die Herstellung von Fruchttabletten beschrieben. Dafür wird Macaubapulpe mit Maltodextrin und Wasser gemischt und die Mischung in rechteckige Form gebracht. Nach der Gefriertrocknung wird eine Fruchttablette erhalten. Somit gibt keines dieser Dokumente einen Hinweis, ob aus Macaubapulpe sensorisch ansprechende, helle und funktionelle Ballaststoffpräparate für Lebensmittel, Heimtiernahrung, Tiernahrung, Kosmetik und technischen Anwendungen gewonnen werden können.

### Aufgabe der vorliegenden Erfindung

Die Aufgabe der vorliegenden Erfindung war es, Ballaststoffpräparate mit guten technofunktionellen Eigenschaften und ansprechenden sensorischen Eigenschaften wie einer hellen Farbe und einem neutralen Geschmack kostengünstig und ohne chemische Modifikation bereit zu stellen und damit die bestehenden Nachteile des Standes der Technik zu umgehen. Die Ballaststoffpräparate sollen sich insbesondere für die Herstellung von Lebensmitteln, Heimtiernahrung und für technische Applikationen eignen.

### Beschreibung der Erfindung

Gelöst wird die Aufgabe durch das Ballaststoffpräparat und das Verfahren gemäß den Patentansprüchen 1, 7, 18 und 20.

Vorteilhafte Ausbildungen des Ballaststoffpräparates sowie des Verfahrens sind Gegenstand der abhängigen Patentansprüche oder lassen sich der nachfolgenden Beschreibung sowie den Ausführungsbeispielen entnehmen.

In der vorliegenden Patentanmeldung basiert das Ballaststoffkonzept auf seiner umfassenden Definition von CODEX Alimentarius als Kohlenhydratpolymere, die nicht durch die endogenen Enzyme im Dünndarm des Menschen hydrolysiert werden. Insbesondere bezieht sich der Begriff Ballaststoffe in der vorliegenden Patentanmeldung hauptsächlich auf Polysaccharide der Pflanzenzellwand (einschließlich Zellulose, Hemizellulosen, Gummi und Pektine) und Lignin, die gegen Hydrolyse durch Verdauungsenzyme beständig sind und in wässrigen ethanolischen Lösungen mit einer Konzentration von gleicher Konzentration ausgefällt werden oder höher als 78% (v/v). Der Ballaststoffgehalt wird in der vorliegenden Patentanmeldung unter Verwendung der offiziellen Methode der ASSOCIATION OF OFFICIAL ANALYTICAL CHEMISTS (AOAC International) bestimmt, basierend auf dem gravimetrischen Assay nach dem Verdau der Probe mit Verdauungsenzymen, insbesondere α-Amylase, Protease und Amyloglucosidase (Referenzmethode 991.43 von AOAC International).

Bei der vorliegenden Erfindung wurde erkannt, dass Rückstände, die bei der Gewinnung von Pflanzenöl aus der Pulpe von Macaubafrüchten erhalten werden, nach Entfernung eines Teils der alkohol-wasserlöslichen Substanzen einen weitgehend neutralen Geschmack aufweisen und somit direkt ohne weitere Funktionalisierung trotz ihrer geringen Kosten für Rohstoff und Verarbeitung als hochwertiges Ballaststoffpräparat mit sehr guten technofunktionellen und sensorischen Eigenschaften in Lebensmitteln, Heimtiernahrung oder in technischen Applikationen, beispielsweise für alle in der Beschreibungseinleitung beim Stand der Technik angegebenen Anwendungen, genutzt werden können.

Das erfindungsgemäße Präparat hat einen Ölgehalt von kleiner 20 Massen-%, vorteilhaft kleiner 10 Massen-%, besser kleiner 5 Massen-%, besonders vorteilhaft kleiner 3 Massen-%, noch vorteilhafter kleiner 2 Massen-%. Der Wassergehalt im Präparat liegt zur Sicherung der mikrobiologischen und sensorischen Eigenschaften bei Werten kleiner 20 Massen-%, vorteilhaft kleiner 15 Massen-%, besonders vorteilhaft kleiner 10 Massen-%, besser kleiner 7 Massen-%. Nach Absenkung des Wassergehalts auf eine geringe Feuchte ist weitgehend sichergestellt, dass die Hydrolyse und Oxidation des im erfindungsgemäßen Präparat enthaltenen Öls langsam verläuft und so die Bildung von geruchs- und geschmacksaktiven Verbindungen vermieden wird.

Besondere Vorteile weist das erfindungsgemäße Präparat auf, wenn der Anteil an alkohol-wasserlöslichen Substanzen (AWS) bezogen auf die Masse des Präparates unter 40% liegt. Als alkohol-wasserlösliche Stoffe werden im Folgenden alle Verbindungen verstanden, die in Ethanol-Wasser-Gemischen mit einem Massenanteil von 80% Ethanol und bei einer Temperatur von 80 °C löslich sind. Dies sind neben anderen löslichen Verbindungen insbesondere Zucker, darunter Mono-, Di- und Oligosaccharide mit bis zu 10 Monomereinheiten.

Das erfindungsgemäße Produkt ist besonders hell und weist umso bessere funktionelle Eigenschaften auf, wenn der Anteil an alkohol-wasserlöslichen Substanzen im Präparat kleiner 35 Massen-% ist, vorteilhaft kleiner 30 Massen-%, besonders vorteilhaft kleiner 20 Massen-%, noch besser kleiner 10 Massen-%, am besten kleiner 5 Massen-%. Durch eine weitestgehende Abtrennung der alkohol-wasserlöslichen Substanzen kann die Funktionalität des Ballaststoffpräparates und die Applikationsbreite gesteigert werden.

Überraschenderweise verbessern sich bei reduziertem Gehalt an alkohol-wasserlöslichen Substanzen, die ja zum größten Teil aus Zuckern bestehen, die sensorischen Eigenschaften deutlich. Aus dem Stand der Technik ist hingegen bekannt, dass Zucker die sensorischen Eigenschaften verbessern. Zudem schmecken die Präparate bei reduziertem Gehalt an alkohol-wasserlöslichen Substanzen neutraler, die Farbe ist heller und auch die Braunfärbung bei Erhitzung im Ofen oder Extruder fällt geringer aus, je weniger alkohol-wasserlösliche Substanzen im erfindungsgemäßen Präparat enthalten sind. Dies ist besonders vorteilhaft für Anwendungen, die eine Erhitzung erfordern, wie erhitzte Lebensmittel oder Polymere, die im Extruder ausgeformt werden müssen.

Wesentliche Eigenschaften des erfindungsgemäßen Ballaststoffpräparats aus der Pulpe von Macaubafrüchten sind die folgenden (Massen- und Prozentangaben sind auf Trockensubstanz bezogen):
- Ballaststoffgehalt höher als 25 Massen-%, besser höher als 30 Massen-%, noch besser höher als 40 Massen-%, vorteilhaft höher als 50 Massen-%, besonders vorteilhaft höher als 60 Massen-%;
- Fettgehalt kleiner 20 Massen-%, kleiner 10 Massen-%, vorteilhaft kleiner 5 Massen-% besonders vorteilhaft kleiner 3 Massen-%;
- Helle Farbe, bestimmt gemäß CIE-L*a*b*-Farbmessung mit einen L*-Wert größer 84, vorteilhaft größer 90, besonders vorteilhaft größer 95;
- Anteil an alkohol-wasserlöslichen Substanzen am Ballaststoffpräparat bezogen auf die Masse des Präparates weniger als 40%;

Das Präparat weist außerdem mindestens eine funktionelle Eigenschaft auf, bessere mehrere, wie z.B. Emulgierung und/oder Schaumbildung und/oder Wasser- und/oder Ölbindung. Diese funktionellen Eigenschaften sind weiter unten detailliert beschrieben.

Die funktionellen Eigenschaften können weiter verbessert werden, wenn die Partikelgrößenverteilung der Ballaststoffpräparate zum Beispiel durch Vermahlen in einen bestimmten Bereich eingestellt wird. Besonders gut dosierbar und gleichzeitig funktionell sind die Präparate mit einer D₉₀-Partikelgröße kleiner 1 mm (D₉₀-Wert: 90% des Volumens der Partikel sind kleiner als 1 mm), vorteilhaft kleiner 500 µm, besonders vorteilhaft kleiner 250 µm. In einigen Fällen kann die Funktionalität bei D₉₀-Werten kleiner 100 µm noch weiter gesteigert werden, weshalb ein Präparat mit dieser Partikelgrößenverteilung vorteilhaft ist. Beim Vergleich der Ballaststoffpräparate fällt auf, dass die Dosierung bei etwas höheren Fettgehalten einfacher ist, da der Staubanteil geringer ausfällt. Ein Fettgehalt kleiner 1 Massen-% ist bei D₉₀-Werten kleiner 250 µm für das Dosieren nicht so vorteilhaft wie ein Fettgehalt zwischen 2 Massen-% und 5 Massen-%. Daher ist es vorteilhaft, die Ballaststoffpräparate nicht unter 1 Massen-% zu entölen. Dies kann u.a. durch Verwendung weniger lipophiler Lösemittel wie z.B. Ethanol oder Propanol anstelle von Hexan erreicht werden.

Nach der Behandlung des Ballaststoffpräparats mit Lösungsmitteln muss der Lösungsmittelanteil reduziert werden. Hierbei kommen Temperaturen von 25 bis 120 °C zum Einsatz, bevorzugt größer 80 °C, vorteilhaft größer 100 °C, und Drücke kleiner 1 bar, vorteilhaft kleiner 500 mbar, besonders vorteilhaft kleiner 200 mbar.

Überraschenderweise zeigen Ballaststoffpräparate, die noch einen geringen Anteil Lösemittel wie Hexan oder Alkohol enthalten, Vorteile hinsichtlich Löslichkeit und anderer funktioneller Eigenschaften im Vergleich zu Lösemittel-freien Präparaten. In einer vorteilhaften Ausgestaltung enthält das Präparat daher organische Lösemittel im Bereich von 1 bis 8000 ppm, vorteilhaft zwischen 10 und 100 ppm. Bei Einsatz von Alkoholen zeigen sich in einigen Fällen Vorteile bei Gehalten an Ethanol oder Propanol größer 1000 ppm bis 8000 ppm.

Im Folgenden wird bei Ballaststoffzubereitungen aus dem Fruchtfleisch von Macaubafrüchten zwischen Mehlen (zum Vergleich) und Konzentraten unterschieden. Als Mehle werden in der vorliegenden Patentanmeldung die Produkte bezeichnet, die direkt nach der Entölung erhalten werden, also noch einen Anteil von ≥ 40 Massen-% an alkohol-wasserlöslichen Substanzen enthalten. Als Konzentrat wird eines der erfindungsgemäßen Ballaststoffpräparate bezeichnet, das weniger alkohol-wasserlösliche Stoffe aufweist, und zwar weniger als 40 Massen-%, besser weniger als 35 Massen-%, noch besser weniger als 30 Massen-%, vorzugsweise weniger als 20 Massen-%, besonders vorteilhaft weniger als 10 Massen-% oder sogar weniger als 5 Massen-%.

In vorteilhaften Ausführungsformen kann die Verwendung von Verarbeitungshilfsstoffen zu einer helleren Farbe und neutralen sensorischen Eigenschaften der Macauba-Ballastoffe beitragen. Dazu gehört beispielsweise die Verwendung von Säuren wie Zitronensäure, Essigsäure, Maleinsäure und Antioxidantien wie Ascorbinsäure, Cystein und Natriumbisulfit bei der Reduktion von alkohol- und wasserlöslichen Substanzen. Darüber hinaus kann eine Reduktion des Sauerstoffgehalts durch Anwendung von Inertgasen wie Stickstoff direkt im Produkt und/oder in der Atmosphäre der Verarbeitungsanlage erfolgen, wodurch die Oxidation von Lipiden und sekundären Pflanzenstoffen verhindert wird. Der Einsatz von Säuren, Antioxidantien und Inertgas kann separat oder in Kombination in einem Prozessschritt, besser in mehreren Prozessschritten, erfolgen.

Die Autoren der vorliegenden Anmeldung haben gefunden, dass auch die Art der Macauba-Pflanze einen großen Einfluss auf die Eigenschaften der Präparate hat. Ballaststoffpräparate aus den Früchten von Acrocomia aculeata unterscheiden sich zum Teil signifikant in einigen funktionellen Eigenschaften und in der Farbe von z.B. Acrocomia totai oder anderen Arten. Präparate aus A. aculeata eignen sich beispielsweise besonders gut als Emulgator und zeigen vielfach einen höheren Emulgieraktivitätsindex als A. totai. So weisen Ballaststoffmehle aus A. aculeata einen Emulgieraktivitätsindex von größer 30 m²/g trocknem Präparat auf und zeigen eine Emulsionsstabilität von größer 5 Minuten, und eine Emulgierkapazität von größer als 400 ml/g trocknem Präparat. Ballaststoffkonzentrate aus A. aculeata weisen einen Emulgieraktivitätsindex von größer 35 m²/g trocknem Präparat auf, besser größer 55 m²/g trocknem Präparat, besonders vorteilhaft größer 70 m²/g trocknem Präparat und erzeugen Emulsionen mit einer Emulsionsstabilität von größer 30 Minuten, vorteilhaft größer 60 Minuten besonders vorteilhaft größer 120 Minuten. Ballaststoffkonzentrate aus A. aculeata weisen eine Emulgierkapazität von größer 500 mL/g trocknem Präparat auf, besser größer 600 mL/g trocknem Präparat, besonders größer 700 mL/g trocknem Präparat. Die Emulgiereigenschaften der Ballaststoffpräparate können durch Zusatz von 10 bis 90 Massen-% an Macaubaproteinmehl oder an anderen Pflanzenproteinpräparaten (%-Anteil bezogen auf den durch die Zugabe erhaltenen Proteinanteil in der Mischung aus Ballaststoffpräparat und Protein), vorteilhaft 20-80 Massen%, besonders vorteilhaft 40-60 Massen-% deutlich verbessert werden.

Ballaststoffpräparate aus den Früchten von A. totai eignen sich ebenfalls als Emulgatoren. Zwar erreichen sie nur geringere Werte für den Emulgieraktivitätsindex, allerdings sind die Emulgierstabilitäten vielfach höher als bei A. aculeata. So weisen Ballaststoffmehle aus A. totai einen Emulgieraktivitätsindex von größer 25 m²/g trocknem Präparat auf und haben eine Emulsionsstabilität von größer 30 Minuten, und eine Emulgierkapazität von größer 300 mL/g trocknem Präparat auf. Ballaststoffkonzentrate aus Acrocomia totai weisen einen Emulgieraktivitätsindex von größer 25 m²/g trocknem Präparat auf, besser größer 35 m²/g trocknem Präparat, besonders vorteilhaft größer 45 m²/g trocknem Präparat auf und erzeugen Emulsionen mit einer Emulsionsstabilität von größer 30 Minuten, vorteilhaft größer 60 Minuten besonders vorteilhaft größer 120 Minuten, in Einzelfällen größer 180 Minuten. Ballaststoffkonzentrate aus A. totai weisen eine Emulgierkapazität von größer 500 mL/g trocknem Präparat auf, besser größer 600 mL/g trocknem Präparat, besonders größer 700 mL/g trocknem Präparat.

Ballaststoffpräparate aus den Früchten von A. aculeata zeigen auch Schaumbildungseigenschaften. So weisen Ballaststoffmehle aus A. aculeata eine Schaumaktivität größer 100 Vol-% auf und erreichen eine Schaumstabilität von größer 5 Vol-%. Ballaststoffkonzentrate aus A. aculeata zeigen deutlich bessere Schaumbildungseigenschaften. Sie erreichen Werte für die Schaumaktivität größer 200 Vol-%, vorteilhaft größer 400 Vol%, besonders vorteilhaft größer 600 Vol-% und erreichen eine Schaumstabilität von größer 25 Vol-%, vorteilhaft größer 50 Vol.-%, besonders vorteilhaft größer 75 Vol.-%, in Einzelfällen sogar größer 90 Vol-%.

Ballaststoffpräparate aus den Früchten von A. totai zeigen bereits als Mehle besonders gute Schaumbildungseigenschaften, also auch ohne vorherige Abtrennung der alkohol-wasser-löslichen Substanzen. So weisen Ballaststoffmehle aus A. totai eine Schaumaktivität größer 200 Vol-% auf und erreichen eine Schaumstabilität von größer 25 Vol-%. Die Eigenschaften der Schaumbildung sind bei Ballaststoffkonzentraten aus A. totai vergleichbar, allerdings sind die Konzentrate nochmal heller in der Farbe. Sie erreichen Werte für die Schaumaktivität größer 200 Vol-% auf, besser größer 400 Vol-%, besonders vorteilhaft größer 600 Vol-%, in einigen Fällen größer 700 Vol-% und erreichen eine Schaumstabilität von größer 25 Vol-%, vorteilhaft größer 50 Vol.-%, besonders vorteilhaft größer 75 Vol.-%, in Einzelfällen sogar größer 85 Vol-%. Damit kommen diese Präparate in ihren Eigenschaften und in der Farbe Eiklar sehr nahe, so dass eine Nutzung der erfindungsgemäßen Präparate in pflanzlichen Substituten für Eiklar möglich wird.

Auch die Werte der Öl- und die Wasserbindung der erfindungsgemäßen Präparate sind im Vergleich zu anderen Präparaten aus anderen Rohstoffen sehr hoch. Auch bei diesen Parametern zeigt sich, dass Konzentrate meist bessere Eigenschaften aufweisen. Die Wasserbindekapazität von Ballaststoffmehlen aus A. acuelata ist größer als 1 mL/g TS (g TS: bezogen auf ein Gramm in Trockensubstanz). Die Wasserbindekapazität bei Ballaststoffkonzentraten aus A. acuelata ist größer als 1,5 mL/g TS, vorteilhaft größer 3 mL/g TS, besonders vorteilhaft größer 4 mL/g TS. Die Wasserbindekapazität von Ballaststoffmehlen aus A. totai sind vergleichbar. Mehle zeigen Werte größer als 1 mL/g TS. Konzentrate aus A. totai haben eine Wasserbindung größer als 1,5 mL/g TS, vorteilhaft größer 3 mL/g TS, besonders vorteilhaft größer 4 mL/g TS.

Die Ölbindekapazität von Ballaststoffmehlen aus A. acuelata ist größer als 1 mL/g TS. Die Ölbindekapazität von Ballaststoffkonzentraten aus A. acuelata ist größer als 1,5 mL/g TS, vorteilhaft größer 3,5 mL/g TS, besonders vorteilhaft größer 5 mL/g TS. Die Ölbindekapazität bei Ballaststoffmehlen aus A. totai ist größer als 0,8 mL/g TS. Die Ölbindekapazität bei Konzentraten aus A. totai ist größer als 1,5 mL/g TS, vorteilhaft größer 3,5 mL/g TS, besonders vorteilhaft größer 4,5 mL/g TS.

Damit zeigt sich die sehr gute Eignung der erfindungsgemäßen Ballaststoffpräparate aus Macaubapulpe für verschiedene Anwendungen in Lebensmitteln besonders aufgrund der hellen Farbe, der neutralen sensorischen Eigenschaften und der guten Funktionalität. Durch Entölung von Pulpe aus Macaubafrüchten und Reduzierung des Anteils an alkohol-wasserlöslichen Substanzen gelingt somit auf einfache Weise die Bereitstellung eines natürlichen und funktionellen Verdickungsmittels oder Ballaststoffes für Anwendungen in Lebensmitteln z.B. zur Herstellung von Schäumen (Kuchen, Füllungen, Backwaren) oder Emulsionen (Cremes, Mayonnaise, Saucen, Süßwaren, Feinkostartikel) und vielen anderen. Auch für den Einsatz in Heimtiernahrung eignen sich die erfindungsgemäßen Konzentrate. Anders als bei konventionellem Tierfutter ist bei Tiernahrung für Hunde, Katzen oder andere Haustiere mit sehr gutem Geruchssinn auf einen besonders neutralen Geruch und Geschmack zu achten, besonders im Hinblick auf typisch pflanzliche grüne, grasige, bohnige und bittere oder adstingierende Geruchs- und Geschmacksnoten. Dies sensorisch neutralen Attribute werden mit den erfindungsgemäßen Präparaten erreicht.

Besonders vorteilhaft für Schäume und Gele sind aufgrund der erreichbaren Schaumvolumina und der hellen Farbe Präparate aus A. totai. Somit kann auch durch die Auswahl der Art oder auch der Varietät der Macauba Pflanze ein besonders passendes Profil hinsichtlich Funktionalität und Sensorik erhalten werden.

Je nach Wahl des Reifegrads der Früchte oder der ausgewählten Art der Macaubapflanze (z.B. A. aculeata oder A. totai) wird nach Abtrennung des Öls aus der Pulpe und nach Reduktion des Gehaltes an alkohol-wasserlöslichen Substanzen ein Ballaststoffpräparat erhalten, dass eine hellgelbe bis nahezu weiße Farbe aufweist, neutral schmeckt und sehr gute technofunktionelle Eigenschaften hat. Damit erfindungsgemäße Präparate je nach Reifegrad, Art, Varietät und Wachstumsbedingungen sehr speziell für unterschiedliche Anwendungen ausgewählt und an Applikationen angepasst werden. Trotz der sehr geringen Kosten stehen damit sehr hochwertige Präparate für unterschiedliche Industriezweige zur Verfügung.

In besonders vorteilhaften Ausgestaltungen der Erfindung wird das Konzentrat in bis zu sechs weitere Fraktionen aufgetrennt, womit besonders funktionelle und wertvolle Fraktionen als Ballaststoffpräparate erhalten werden. Diese sind: (1) eine wasserlösliche Fraktion (löslich in Wasser zwischen 5 und 100 °C) und eine wasserunlösliche Restfraktion (2). Die Fraktion (2) kann unter Verwendung eines alkalischen und chelatbildenden Extraktionsmilieus in eine zweite lösliche Pektinfraktion (3) und eine unlösliche Fraktion (4) getrennt werden. Dazu werden 0,05-0,1 mol/L NaOH oder Natriumcarbonat verwendet, um einen milden alkalischen Zustand zu gewährleisten, und 0,5 mmol EDTA oder CDTA oder 0,5% (m/v) Ammoniumoxalat für die Chelatisierungsaktivität.

Die Fraktion (4) kann wiederum mit Hilfe von hochkonzentrierter Kalilauge (1-4 mol/L), optional unter Zusatz von 10 bis 50 mmol Natriumborhydrid in eine lösliche Hemizellulose-Fraktion (5) und einen unlöslichen zellulosereichen Rückstand (6) aufgetrennt werden. Diese Fraktionen zeigen ganz unterschiedliche Wasser- und Ölbindeeigenschaften. So ist Fraktion (1) besonders gut und transparent in Wasser löslich, bildet feste Gele aus und ermöglichst es, dass sich nach dem Trocknen des Wassers - auch ohne den Zusatz von Weichmachern oder Vernetzern - reißfeste und flexible Filme ausbilden. Je weniger alkohol-wasserlösliche Substanzen in Fraktion (1) enthalten sind, desto besser sind die Eigenschaften der Filme. Daher wird man bemüht sein, den Anteil an alkohol-wasserlöslichen Zuckern in der Fraktion (1) auf Werte kleiner 40 Massen-% zu reduzieren, vorteilhaft kleiner 30 Massen-%, besser kleiner 20 Massen-%, besonders vorteilhaft kleiner 10 Massen-%, noch besser kleiner 5 Massen-%. Es zeigt sich, dass Filme, die mit der Fraktion (1) hergestellt werden, mit abnehmendem Gehalt an alkohol-wasserlöslichen Substanzen transparenter und fester werden als Filme mit hohen Gehalten an alkohol-wasserlöslichen Substanzen. Damit bietet sich die Fraktion (1) neben anderen Applikationen sowohl für Anwendungen von Schäumen und Gelen in Lebensmitteln, Kosmetika und Heimtiernahrung an, als auch für technische Filme, Beschichtungen, Klebstoffe und andere. Hinsichtlich der funktionellen Eigenschaften zeichnet sich die Fraktion (1) aufgrund ihrer guten Wasserlöslichkeit durch eine sehr kleine bis nicht messbare Wasserbindung und eine nur schwach ausgeprägte Ölbindung aus, die vorteilhaft zwischen 0,5 und 2 mL Öl/g TS liegt. Dieser Wert für die Ölbindung zeichnet auch die Eigenschaft der Fraktionen (3) und (5) aus. Eine besonders gute Ölbindung mit Werten größer 5 mL Öl/g TS bis größer 8 mL Öl/g TS zeigt hingegen Fraktion (6).

Besonders gute Eigenschaften hinsichtlich der Wasserbindung zeigen die in den Fraktionierungsschritten anfallenden unlöslichen Fraktionen. Hier steigt die Wasserbindung von Macaubamehl und -konzentrat mit Werten größer 1 bis größer 3 mL/g TS, auf Werte von größer 4 bis größer 8 mL/g TS in den Fraktionen (2), (4), (5) und (6). Nur die Fraktion (3) zeigt mit kleiner 2 mL/g TS eine nicht so ausgeprägte Wasserbindung.

Als Anwendungen für alle obigen Fraktionen bieten sich wie bei dem Konzentrat alle Applikationen an, bei denen Funktionalitäten v.a. im Zusammenspiel mit Wasser benötigt werden. Dies gilt für alle Arten von Lebensmitteln, Kosmetikprodukte und Heimtiernahrung oder für essbare oder nicht-essbare Beschichtungen und Filmen auf Lebensmitteln, z.B. zur Verlängerung der Haltbarkeit. Bei Anwendungen in Lebensmitteln fällt die Ähnlichkeit von Macaubakonzentrat zu Guarkernmehl und Johannisbrotkernmehl auf, da das Verhalten im Bereich der Rheologie, der Gelbildung und Verdickung vielfach vergleichbar ist.

Geeignet sind die erfindungsgemäßen Präparate auch für technische Hilfsstoffe. Dies können Emulgatoren für Farbe und Lacke, technische Schaumbildner, technische Filme, Folien und Beschichtungen, Klebstoffe, Schmierstoffe oder Bohrflüssigkeiten in der Erdölförderung sein. Besonders in diesem Sektor der Gewinnung fossiler Ressourcen wird zunehmend auf umweltverträgliche Verdicker geachtet, eine Forderung, die mit Macauba-Präparaten sehr gut zu realisieren ist.

Im Folgenden wird das erfindungsgemäße Verfahren zur Herstellung der Ballaststoffpräparate beschrieben. Das Verfahren weist wenigstens die folgenden Schritte auf:
- Bereitstellen von teilentölter Pulpe aus Macaubafrüchten, vorzugsweise von Acrocomia acuelata und/oder Acrocomia totai, mit einem Fettgehalt zwischen 3 Massen-% und 60 Massen-% bezogen auf Trockenmasse. Die Fettgehalte der teilweise entölten Pulpe können je nach Pflanzenart oder Erntezeitpunkt variieren oder sie unterscheiden sich je nach Vorbehandlung (wie z.B. Vorpressen, Trocknen, Flockieren, mechanisches Pressen, andere konventionelle Verfahren zur Pflanzenölgewinnung).
- Reduktion des Ölgehaltes in der Pulpe auf Werte (in TS) kleiner 20 Massen-%, vorzugsweise kleiner 10 Massen-%, besser kleiner 5 Massen-%, besonders vorteilhaft kleiner 3 Massen-%, noch besser kleiner 2 Massen-% mit Hilfe extraktiver Verfahren.
- Reduktion des Gehaltes an alkohol-wasserlöslichen Substanzen in der Pulpe auf Werte (in TS) kleiner 40 Massen-%, besser kleiner 35 Massen-%, bevorzugt kleiner 30 Massen-%, vorteilhaft kleiner 20 Massen-%, besonders vorteilhaft kleiner 10 Massen-%, noch besser kleiner 5 Massen-%, mit Hilfe extraktiver Fraktionierungsverfahren. Dabei wird das Pulpenkonzentrat erhalten. Vorteilhaft kommen Fest-Flüssig-Extraktionsverfahren zum Einsatz (z.B. in der Ausführung als Mischreaktor, Perkolation, Gegenstromextraktion usw.). Trockenfraktionierungstechniken wie Mahlen, Sieben und Windsichtung können ebenfalls angewendet werden.

Optional und vorteilhaft:
- Die Reduktion des Ölgehaltes erfolgt mit Hilfe von Lösemitteln. Als Lösemittel zum Einsatz kommen können dabei beispielsweise Hexan, Ethanol, Propanol, überkritisches CO₂ oder andere unter- oder überkritische Lösemittel und weitere organische Lösemittel.
- Nutzung von Mischungen aus Ethanol und Wasser im Massenverhältnis von 94:6 bis 90:10 (jeweils Ethanol zu Wasser) bei Temperaturen von 40-70°C, vorteilhaft 50-65°C zur simultanen Abtrennung von Öl und löslichen Substanzen aus der Macaubapulpe.
- Weitere Reduktion des Ölgehaltes und Reduktion des Gehaltes an alkohol-wasserlöslichen Substanzen in der Pulpe durch Nutzung von Wasser oder Mischungen aus Alkohol und Wasser im Massenverhältnis (Alkohol zu Wasser) kleiner 90:10, besser kleiner 80:20 zur Gewinnung des Pulpenkonzentrates. Dabei wird als Alkohol vorzugsweise Propanol oder Ethanol und eine Temperatur zwischen 40 und 90 °C, vorteilhaft zwischen 65 und 85 °C, besonders vorteilhaft von 80 °C verwendet, so dass ein Lösen von alkohol-unlöslichen Kohlenhydraten weitgehend vermieden wird.
- Verwendung von angesäuerten Wasser-Alkohol-Gemischen mit einem pH-Wert im Bereich von 2,0 - 6,0 zur Verringerung des Gehalts an alkohol- und wasserlöslichen Stoffen im Zellstoff. Dies kann durch den Einsatz von Säuren, vorzugsweise milde organische Säuren wie Zitronensäure, Essigsäure oder Maleinsäure, erreicht werden.
- Verwendung von antioxidativen Zusätzen in Wasser-Alkohol-Gemischen zur Senkung des Gehalts an alkoholund wasserlöslichen Stoffen in der Pulpe. Dies kann zum Beispiel mit Ascorbinsäure, Cystein oder Natriumbisulfit geschehen.
- Verringerung des Sauerstoffgehalts im Extraktionsmedium und in der Geräteatmosphäre, um die Oxidation von Öl und sekundären Pflanzenstoffen zu verhindern. Dies kann mit Inert-Gasen wie Stickstoff erfolgen.
- Weitere Reduktion der alkohol-wasserlöslichen Substanzen durch Trockenfraktionierung. Der teilentfettete Macauba-Zellstoff wird optional gemahlen oder direkt weiter verarbeitet. Zum Mahlen kann eine Schneidmühle, Kugelmühle, Prallmühle oder Strahlmühle verwendet werden, der Mahlgrad kann auf eine D90-Volumenpartikelgröße von weniger als 2 mm, vorteilhafterweise weniger als 500 µm, besser weniger als 250 µm, besonders vorteilhaft weniger als 100 µm eingestellt werden. Anschließend wird das Material mit Sieböffnungsdurchmessern von 2 mm bis 50 µm mit 1 bis 10 verschiedenen Sieben gesiebt. Die Windsichtung kann mit verschiedenen Windsichtungsverfahren wie GravitationsGegenstrom, Gravitations-Kreuzstrom, ZentrifugalGegenstrom und Zentrifugal-Kreuzstrom durchgeführt werden.
- Fraktionieren der wasserlöslichen Ballaststofffraktion von den alkohol-wasserlöslichen Substanzen durch folgende Schritte:
   ∘ Extraktion der Pulpe mit einer Mischung aus Alkohol und Wasser, um alkohol-wasserlösliche Substanzen von den wasserlöslichen Ballaststoffen zu separieren, Abtrennen des zuckerhaltigen Extraktes vom Raffinat, dies optional in mehreren Stufen hintereinander und dann Extraktion des Raffinats mit Wasser, vorzugsweise bei Temperaturen über 30°C, vorzugsweise größer 40°C, um die wasserlösliche Ballaststofffraktion zu gewinnen, und/oder
   ∘ Extraktion aller Zucker und wasserlöslicher Ballaststoffe mit Wasser, vorzugsweise bei Temperaturen über 30°C, vorzugsweise größer 40°C, Abtrennung des Extraktes vom Raffinat; gefolgt von der Trennung des Extraktes in eine Fraktion, die in Alkohol-Wasser-Mischungen gut löslich ist und eine Fraktion, die in Alkohol-Wasser-Mischungen nicht gut löslich ist durch Ultrafiltration oder durch alkoholische Fällung von alkohol-wasser-unlöslichen Kohlenhydraten, oder
   ∘ Fraktionierung der wasserlöslichen Ballaststoffe durch Trockenfraktionierung. Das Konzentrat wird optional gemahlen (Schneidmühle, Kugelmühle, Prallmühle oder Strahlmühle) auf eine D90-Volumenpartikelgröße von kleiner 2 mm, vorteilhaft kleiner 500 µm, besser kleiner 250 µm, besonders vorteilhaft kleiner 100 µm oder es wird unvermahlen weiterverarbeitet. Anschließend wird das Material mit Sieböffnungsdurchmessern von 2 mm bis 50 µm mit 1 bis 10 verschiedenen Sieben gesiebt. Die Windsichtung kann mit verschiedenen Windsichtungsverfahren wie Gravitationsgegenstrom, Gravitationsquerstrom, Zentrifugalgegenstrom und Zentrifugalquerstrom durchgeführt werden. Die im trocknen Zustand vorliegenden wasserlöslichen und wasserunlöslichen Fasern werden dabei in verschiedene Fraktionen getrennt.
- Fraktionieren der Ballaststoffe, vorzugsweise nach Entölung auf unter 5 Massen-% und/oder weitgehender Reduktion der alkohol-wasserlöslichen Substanzen durch wässrige Extraktion bei 40°C, durch: Abtrennen eines wässrigen Extraktes von einem Raffinat und vorzugsweise Trocknung der wasserlöslichen Fraktion (1), Extraktion des Raffinats (Fraktion 2) mittels NaOH-EDTA-Lösung mit 0,05-0,1 mol/L NaOH oder Natriumcarbonat und 0,5 mmol EDTA oder CDTA oder 0,5% (m/v) Ammoniumoxalatlösung und Abtrennen und Trocknen der löslichen Fraktion (3) von einem unlöslichen Raffinat (Fraktion 4) und abschließend Extraktion mit konzentrierter Lauge (1-4 mol/L), z.B. Kalilauge, und Abtrennen, Neutralisieren und Trocknen des Extraktes (Fraktion 5) vom unlöslichen Rückstand (Fraktion 6).

Die funktionellen Eigenschaften dieser 6 Fraktionen können nach dem Trocknungsschritt ebenfalls weiter verbessert werden, wenn die Partikelgrößenverteilung der Ballaststoffpräparate durch Vermahlen mit einer Schneid-, Schlag-, Kugeloder Prallmühle in Kombination mit der Nutzung von Sieben und Siebeinsätzen auf einen bestimmten Bereich eingestellt wird. Besonders funktionell sind die Präparate mit einer D₉₀-Partikelgröße kleiner 1 mm, vorteilhaft kleiner 500 µm, besonders vorteilhaft kleiner 250 µm, besser kleiner 100 µm.

Weiterhin zeigt sich vorteilhaft, dass die Eigenschaften der Präparate verbessert werden können, wenn vor dem Trocknen eine definierte Erhitzung erfolgt. Die Temperatur sollte hierbei in einem Bereich zwischen 70 und 120 °C liegen, vorteilhaft zwischen 70 und 100 °C, besonders vorteilhaft zwischen 70 und 80 °C. Die Dauer der Behandlung sollte nicht über 60 Minuten betragen, vorteilhaft kleiner 30 Minuten, besonders vorteilhaft kleiner 15 Minuten.

Im Folgenden wird beschrieben, wie das Bereitstellen der vollfetten oder teilentölten Pulpe erfolgen kann. Nach der Reife der Macaubafrüchte werden diese vorteilhaft ohne Krafteinwirkung vom Fruchtverband abgetrennt, am besten zu unterschiedlichen Zeiten je nach Reifegrad. Bei einer isolierten Ernte einzelner Früchte aus den Fruchtverbänden ist sowohl die Qualität des Öls als auch die der Pulpe am besten. Es ist auch möglich, die Fruchtverbände als Ganzes von der Palme abzuschneiden. Dann sollte vorteilhaft der herabfallende Fruchtverband weich aufgefangen werden, z.B. durch eine weiche Folie oder ein anderes System zum schonenden Abbremsen des Falls, um Beschädigungen der äußeren Schale zu vermeiden.

Vor der weiteren mechanischen Verarbeitung der Früchte sollte vorteilhaft eine thermische Behandlung der Früchte an der Oberfläche erfolgen, auf eine Oberflächentemperatur über 70 °C, vorteilhaft über 75°C, besonders vorteilhaft über 80°C für mindestens 1 Minute (Definition der Dauer: vom Erreichen der Maximaltemperatur bis zum Absinken der Temperatur auf unter 65 °C), vorteilhaft länger als 10 Minuten oder 20 Minuten, besonders vorteilhaft länger als 30 Minuten erfolgen. Im Anschluss sollte vorteilhaft der Wassergehalt der äußeren Schale auf einen Wert kleiner 20 Massen-%, vorteilhaft kleiner 10 Massen-% reduziert werden, um das Schälen effizient zu machen und den Anteil an Pulpe in der Schalenfraktion zu reduzieren. Zum Einsatz kommen kann hier jede bekannte Form der Trocknung. Der Fachmann wird in der Lage sein, je nach gewünschter Qualität des Öls und beabsichtigter Geschwindigkeit der Trocknung aus der Vielfalt der Trocknungsmethoden die geeignete Methode auszuwählen - von der Trocknung im Freien oder an der Sonne, in einer belüfteten oder unbelüfteten Halle oder einem einfachen Umlufttrockner, Kontakt- und Konvektionstrockner bis hin zu einer Vakuumtrocknung.

Als besonders vorteilhaft für eine hohe Ölqualität erweist es sich, wenn nicht nur die Schale getrocknet wird, sondern der Wassergehalt in der ganzen Frucht auf einen Wert unter 20 Massen-%, vorteilhaft kleiner 15 Massen-%, besonders vorteilhaft kleiner 10 Massen-% abgesenkt wird. Besonders nach einer weitgehenden Trocknung auf Werte unter 10 Massen-% sind die Früchte länger haltbar und die Ölqualität wird gesteigert.

Im Anschluss an das Trocknen und einer möglichen Zwischenlagerung erfolgt das Schälen des Exokarps auf einem Schälaggregat nach Stand der Technik. Dabei ist darauf zu achten, dass durch die Wahl der Parameter weniger als 20 Massen-% an Pulpe in der Exokarpfraktion verbleibt, vorteilhaft weniger als 10 Massen-%, besonders vorteilhaft weniger als 5 Massen-% bezogen auf die Masse der Schalenfraktion. Sollte dies in einem Durchlauf nicht erzielbar sein, so ist ein nachfolgender Trennschritt zwischen Epikarp und Pulpe vorzusehen.

Im Ergebnis der Schälung ist überdies darauf zu achten, dass nach dem Schälen keine Schalen oder nur geringe Mengen an Schalen in der Pulpefraktion enthalten sind. Die Schälung ist daher so auszuführen, dass die abgetrennte Pulpe am Ende einen Schalengehalt von weniger als 10 %, besser weniger als 5 %, vorzugsweise weniger als 2 Massen-%, bezogen auf die Trockenmasse aufweist. Der Fachmann aus dem Bereich der Fraktionierung von pflanzlichen Rohstoffen wird in der Lage sein, die geeigneten Aggregate und Prozessparameter für diese Trennaufgabe auszuwählen.

Im folgenden Schritt wird die Pulpe von der inneren harten Schale des Kerns der Steinfrucht, dem Endokarp, abgetrennt. Dies kann mit Schneidmühlen oder anderen Aggregaten durchgeführt werden, die dem Fachmann bekannt sind. Vorteilhaft wird dieser Prozess aus sensorischen Gründen für die erfindungsgemäßen Präparate so gestaltet sein, dass der Anteil an Stückchen aus dem schwarzen Endokarp in der Pulpe kleiner 3 Massen-% liegt, vorteilhaft kleiner 2 Massen-%, besser kleiner 1 Massen-%, besonders vorteilhaft kleiner 0,1 Massen%. Die dabei erhaltene Pulpe wird dem erfindungsgemäßen Verfahren zugeführt. Eine weitere Vorbehandlung kann in einer teilweisen Entölung bestehen. Aufgrund der besonderen Abtrennung von Anteilen des Endokarps aus der Pulpe weisen die im Anschluss mechanisch oder extraktiv gewonnenen Öle einen besonders geringen Anteil an Lignin oder anderen phenolischen Komponenten aus, so dass der Geschmack des Öls neutraler wird.

Die mechanische Entölung wird nach Abtrennen des Wassers aus der Pulpe durch Trocknung auf Werte kleiner 30 Massen-%, besser kleiner 20 Massen-%, vorteilhaft kleiner 15 Massen-%, besonders vorteilhaft kleiner 10 Massen-% vorteilhaft in einer kontinuierlich arbeitenden Presse, z.B. einer Schneckenpresse, einem Extruder oder einer anderen kontinuierlichen oder quasi-kontinuierlichen Pressvorrichtung erfolgen. Der Ölgehalt wird dabei vorteilhaft auf unter 30 Massen-% abgesenkt, besonders vorteilhaft kleiner 20 Massen%, oder kleiner 15 Massen-%. Besonders vorteilhafte technofunktionelle Eigenschaften der erfindungsgemäßen Ballaststoffpräparate werden erhalten, wenn der Ölgehalt nach der mechanischen Entölung zwischen 15 und 25 Massen-% liegt, da eine thermische Schädigung durch zu hohe Reibung vermieden wird.

Nachfolgend werden die Bestimmungsverfahren kurz beschrieben, die zur quantitativen Charakterisierung der hergestellten Ballaststoffpräparate genutzt werden:

### - Ballaststoffgehalt:

Der Ballaststoffgehalt ist definiert als der aus der gravimetrischen Bestimmung Methode nach enzymatischen Aufschluss der Probe abgeleitete Gehalt (Methode 991.43 von AOAC) [8].

### - Proteingehalt:

Der Proteingehalt ist definiert als der Gehalt, der sich aus der Bestimmung des Stickstoffs in einer Probe und der Multiplikation des ermittelten Wertes mit dem Faktor 6,25 errechnet. Der Proteingehalt ist in der vorliegenden Patentanmeldung in Prozent bezogen auf die Trockenmasse (TS) angegeben. Referenzmethoden zur Bestimmung des Proteingehalts sind die Dumas-Verbrennungsmethode [9] und die Kjeldahl-Aufschlussmethode [10].

### - Farbe:

Die wahrnehmbare Farbe ist mittels CIE-L*a*b*- Farbmessung definiert (vgl. DIN 6417). Dabei gibt die L*-Achse die Helligkeit an, wobei Schwarz den Wert 0 und Weiß den Wert 100 hat, die a*-Achse beschreibt den Grün- oder Rotanteil und die b*-Achse den Blau- oder Gelbanteil. Die in der vorliegenden Patentanmeldung angegebenen L*-Werte beziehen sich auf eine Messung bei einer D90-Partikelgröße des Ballaststoffpräparates von 250 µm.

### - Emulgierende Eigenschaften:

Der Emulgieraktivitätsindex und die Emulsionsstabilität werden wie in [11] bestimmt. Die Emulgierkapazität wird nach der in [12] beschriebenen Methode bestimmt. Einer 1 Mass.-%igen Suspension des Ballaststoffpräparats mit einem Volumen von 100 ml wird bei pH 7 Maiskeimöl zugegeben, die Mischung mittels Ultraturrax zu einer Emulsion gerührt und weiterhin Öl zudosiert bis zum Auftreten der Phaseninversion der Öl-in-Wasser-Emulsion. Die Emulgierkapazität ist definiert als das maximale Ölaufnahmevermögen der Suspension aus Wasser und Ballaststoffpräparat, bestimmt über die spontane Abnahme der Leitfähigkeit bei der Phaseninversion. Das Emulgiervermögen wird in ml Öl/g Ballaststoffpräparat angegeben, also Milliliter emulgiertes Öl pro Gramm Ballaststoffpräparat.

### - Fettgehalt:

Der Fettgehalt wird gravimetrisch mit der Sohxlet-Methode [13] bestimmt (Methode 920.39 von AOAC).

### - Schaumaktivität:

Die Schaumaktivität ist angegeben in Prozent, gemessen als Volumenzunahme einer Ballaststofflösung, pH 7, bei Aufschlag wahrend 8 min auf Stufe 3 (591 U/mm) in einer Hobart 50N Standard-Küchenmaschine (Stahlkessel mit 5 Liter Inhalt) mit Rührbesen (Drahtbesen).

### - Schaumstabilität:

Die Schaumstabilität ist angegeben in Prozent, gemessen als übrig gebliebenes Volumen von 100 ml Schaum innerhalb einer Stunde nach Aufschlag entsprechend der Angaben unter Schaumaktivität.

### - Wassergehalt:

Der Wassergehalt wird gravimetrisch bestimmt nach § 64 LFGB-Methoden [14] bei 105 °C bis zur Gewichtskonstanz.

### - Gehalt aus Alkohol-Wasser-löslichen Stoffen:

Gehalt aus Alkohol-Wasser-löslichen Stoffen wird gravimetrisch wie folgt bestimmt: Die Probe (Macauba-Mehl, Ballaststoffpräparat oder Faserfraktion) wird in wässrigem Ethanol 80 % (v/v) im Fest-Flüssig-Verhältnis von 1:10 dispergiert (m/v). Die Dispersion wird unter leichtem Rühren 60 Minuten bei Siedetemperatur (etwa 80 °C) gehalten. Anschließend wird die Mischung zentrifugiert (3300 g, 20 min, 20 oC) und filtriert und der Überstand (Flüssigphase) wird aufbewahrt. Das feste Pellet wird mit 80 % wässrigem Ethanol unter ähnlichen Bedingungen wie oben beschrieben extrahiert, bis ein klarer Extrakt erhalten wird (mindestens 5 Extraktionszyklen). Nach Beendigung der Extraktionszyklen werden die Flüssigextrakte vereinigt, das Ethanol destilliert und das Wasser über Nacht bei 105 °C verdampft. Die nach dem Trocknen verbleibende Feststoffmenge wird gewogen und als % der Probenmenge angegeben, die zu Beginn der Analyse der Extraktion unterzogen wurde.

### - Wasserbindevermögen:

Das Wasserbindevermögen wird nach der Methode der American Association of Cereal Chemists [15] bestimmt.

### -Ölbindevermögen:

Das Ölbindevermögen wird bei Raumtemperatur bestimmt. Die Probe wird in einem Überschuss an Öl dispergiert und nach vollständigem Mischen und Zentrifugieren das nicht vom Produkt gebundene Ölvolumen bestimmt. Die Referenzmethode wird bereitgestellt von [16].

### Ausführungsbeispiele

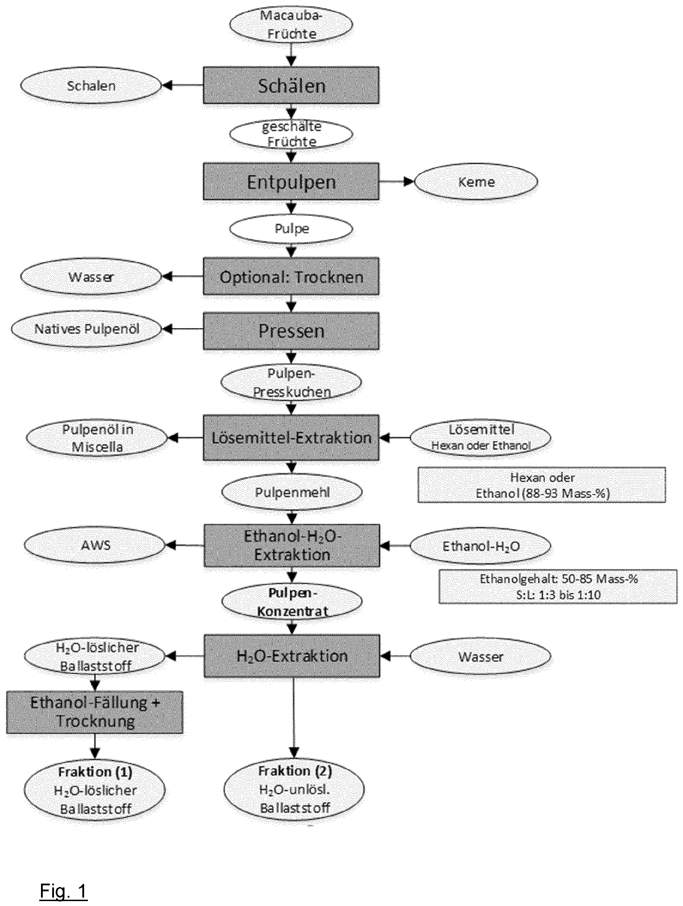

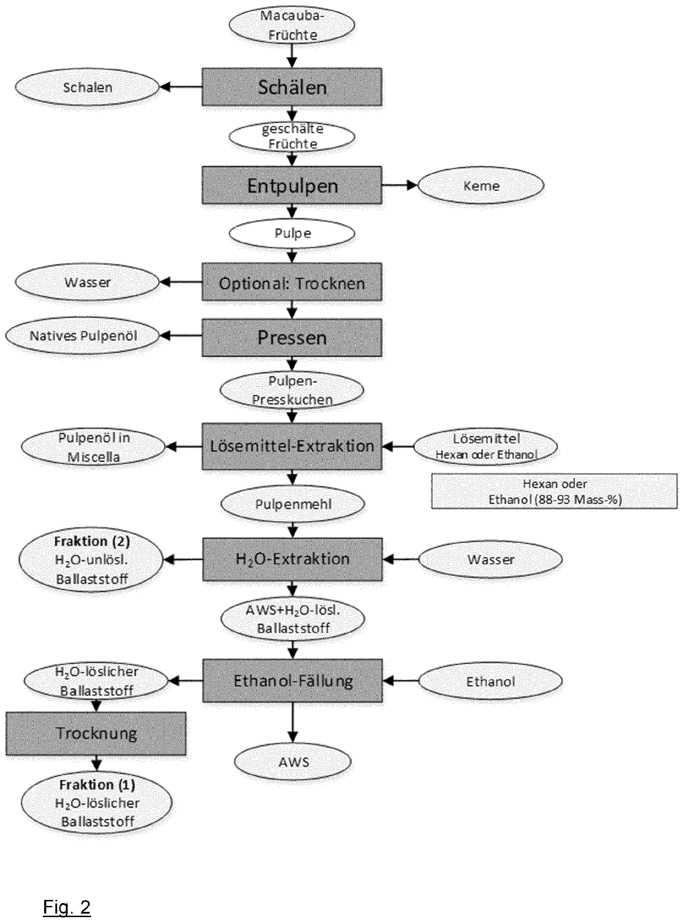

Die Figuren 1 und 2 zeigen beispielhaft zwei Varianten für die Herstellung der vorgeschlagenen Ballaststoffpräparate, wobei in diesen Beispielen auch die Schritte zur Bereitstellung der teilentölten Pulpe dargestellt sind.

### Ausführungsbeispiel 1

### (nur zum Vergleich)

3 Muster von Macaubafrüchten bestehend aus je 20 Früchten pro Muster aus unterschiedlichen Ernteregionen und von unterschiedlichen Arten stammend, wurden manuell von der äußeren Schale (Epicarp) befreit. Anschließend wurde die Pulpe ebenfalls manuell vom inneren Kern (Endocarp) abgetrennt und die dabei erhaltene Pulpe wurde analysiert. Der Wassergehalt lag in den 3 Proben je nach Herkunft, Art und Lagerdauer zwischen 33 und 53 Massen-%. Nach Trocknen der Pulpe bei 45 °C für 12 Stunden im Ofen wurden 3 Proben von nahezu wasserfreier Pulpe ergaben sich hinsichtlich der Zusammensetzung die folgenden Mittelwerte (Tabelle 1):

**Tabelle 1: Mittlere Zusammensetzung von Macauba-Pulpe aus 3 verschiedenen Rohstoffen (TS: bezogen auf Trockensubstanz)**

| **Komponente** | **Mittelwert +/-** |
|---|---|
| Fett (Massen-% TS) | 46.1 - 59,3 |
| Protein (Massen-% TS) | 1,2 - 4,8 |
| Asche (Massen-% TS) | 1,6 - 3,3 |
| Ballaststoff (Massen-% TS) | 17,0-27,0 |
| Kohlenhydrate (Massen-% TS) | 20,1-36,2 |
| Zucker (Massen-% TS) | 10,6 - 32,8 |

### Ausführungsbeispiel 2

Zwei Proben von je 500 g Pulpe von A. aculeata und A. totai, gewonnen wie in Beispiel 1, wurden in 2 Präparate fraktioniert. Eine Fraktion (Pulpenmehl) wurde erhalten durch Pressung der getrockneten Pulpe auf einen Ölgehalt von 20 Massen-% gefolgt von einer Entölung mit reinem Ethanol im Soxhlet-Apparat für 12 Stunden (Mehl). Im Anschluss wurde die entölte Probe geteilt, ein Teil wurde direkt analysiert, der andere Teil wurde entsprechend der vorliegenden Erfindung mit einer Mischung aus 20% Wasser und 80 % Ethanol im Massenverhältnis bei 80 °C mehrfach extrahiert, wobei in jeder Stufe der Extrakt vom Raffinat getrennt wurde und frisches Lösemittel zugesetzt wurde. Am Ende entsprach die Masse des Pulpenkonzentrats bezogen auf TS rund 60 % der Masse des eingesetzten Mehls. Es ergaben sich die in Tabelle 2 angegebenen Zusammensetzungen und die in Tabelle 3 angegebenen funktionellen Eigenschaften der Fraktionen. Durch die Behandlung mit einer Mischung aus Ethanol und Wasser konnte der Anteil an Ballaststoffen von 40,5 Massen-% auf über 80 Massen-% deutlich erhöht und der (aus Differenzmessung ermittelte) Anteil an Kohlenhydraten von über 50 Massen-% auf 10,6 Massen-% abgesenkt werden.

**Tabelle 2: Zusammensetzung von entölter Macauba Pulpenmehl (MPM) und Pulpenkonzentrat (MPK) aus (Acrocomia aculeata) bezogen auf Trockensubstanz (TS)**

| **Komponente** | **Zusammensetzung (Massen- % in TS)** | |
|---|---|---|
| | **MPM** | **MPK** |
| Ballaststoffe | 40.5 | 80.5 |
| Protein | 3.9 | 6.3 |
| Fett | 1.0 | <0.5 |
| Asche | 3.6 | 2.1 |
| AWS | 51.5 | 10.6 |

**Tabelle 3: Funktionelle Eigenschaften von Pulpenmehl (MPM) und Pulpen-Konzentrat (MPK) bezogen auf TS**

| **Funktionelle Eigenschaften** | ***A. acuelata*** | | ***A. totai*** | |
|---|---|---|---|---|
| | **Mehl** | **Konzentrat** | **Mehl** | **Konzentrat** |
| Wasserbindekapazität (mL/g TS) | 3.68 ± 0.03 | 4.61 ± 0.04 | 3.32 ± 0.11 | 4.71 ± 0.26 |
| Ölbindekapazität (mL/g TS) | 3.50 ± 0.05 | 5.41 ± 0.18 | 2.53 ± 0.25 | 5.03 ± 0.25 |
| Schaumaktivität (Vol-%) | 234.5 ± 26.7 | 685.9 ± 7.3 | 731.3 ± 0.1 | 798.4 ± 22.2 |
| Schaumstabilität (Vol-%) | 11.5 ± 4.4 | 93.3 ± 2.2 | 88.3 ± 5.5 | 86.7 ± 2.2 |
| Emulgieraktivitätsindex (m²/g TS) | 68.9 ± 1.4 | 75.5 ± 13.2 | 62.0 ± 3.4 | 49.7 ± 2.2 |
| Emulsionsstabilität (min) | 27.2 ± 5.3 | 157.6 ± 23.0 | 164.6 ± 6.2 | 242.5 ± 57.0 |
| Emulgierkapazität (mL/g TS) | 420 ± 25 | 798 ± 19 | 350 ± 3 | 685 ± 3 |
| L*-wert | Nicht gemessen | Nicht gemessen | 90,47 ± 0,07 | 89,70 ± 0,08 |

### Ausführungsbeispiel 3

Aus einer Probe von 500 g Pulpenkonzentrat aus A. aculeata, gewonnen wie in Beispiel 2, wurde entsprechend der vorliegenden Erfindung fraktioniert. Dafür wurden 5000 mL Wasser bei 40 °C dem Konzentrat zugesetzt, die Mischung wurde 30 Minuten gerührt und dann der unlösliche Anteil vom löslichen Anteil durch Filtration abgetrennt. Der Vorgang wurde 3-mal wiederholt. Die erhaltene lösliche Fraktion (1) wurde getrocknet und analysenfein vermahlen. Der Rückstand wurde mit einer 0,05 mol/L NaOH-Lösung der 0,5 mmol/L EDTA zugesetzt wurde dreimal extrahiert. Als lösliche Fraktion wurde eine Pektin-reiche Fraktion (3) erhalten, getrocknet und vermahlen. Im dritten Schritt wurde der Rückstand (Fraktion 4) mit konzentrierter Kalilauge 2 mol/L erneut dreimal extrahiert, der lösliche Überstand (Fraktion 5) wurde vom unlöslichen Rückstand (Fraktion 6) getrennt. Alle Fraktionen wurden getrocknet und vermahlen. Von den erhaltenen Proben wurden die Wasser- und Ölbindung (Tabelle 4) sowie die Fließeigenschaften (Tabelle 5) bestimmt.

**Tabelle 4: Funktionelle Eigenschaften von Fraktionen aus Macauba Pulpe (A. acuelata)**

| **Macauba Pulpen Fraktion** | **Wasserbindung (mL/g TS)** | **Ölbindung (mL/g TS)** |
|---|---|---|
| Macauba Pulpenmehl | 3.7 ± 0.0 | 3.5 ± 0.0 |
| Wasserlösliche Fraktion (1) | Nicht bestimmbar | 1.1 ± 0.1 |
| Fraktion (3) | 1.3 ± 0.3 | 1.1 ± 0.2 |
| Fraktion (5) | 4.6 ± 0.2 | 1.1 ± 0.3 |
| Unlösliche Fraktion (6) | 8.8 ± 0.1 | 8.2 ± 0.3 |

Hinsichtlich der Wasser- und Ölbindung zeigt sich, dass durch Fraktionierung des Pulpenkonzentrats eine gezielte Anpassung der funktionellen Eigenschaften möglich wird. So zeigt die unlösliche Fraktion (6) ähnlich wie die in Tabelle 4 nicht dargestellten Fraktionen (2) und (4) sehr gute Bindungseigenschaften sowohl hinsichtlich der Wasserbindung als auch hinsichtlich der Ölbindung. Die Ölbindung hingegen ist bei den Fraktionen (1), (3) und (5) mit etwa 1,1 mL/g TS sehr gering ausgeprägt, die Wasserbindung ist bei den Fraktionen (5) und (6) ebenfalls sehr hoch.

**Tabelle 5: Fließeigenschaften von Fraktionen aus Macauba Pulpe (A. aculeata)**

| **Konzentration (g Präparat/ 100g Lösung)** | **Wasserlösliche Fraktion (1)** | | **Fraktion (3)** | | **Unlösliche Fraktion (6)** | |
|---|---|---|---|---|---|---|
| | **k (mPa ·s)** | **n** | **k (mPa ·s)** | **n** | **k (mPa ·s)** | **n** |
| 0.5 | 19.37 ± 3.60 | 0.92 | 0.13 ± 0.02 | 1.54 | 0.18 ± 0.01 | 1.46 |
| 1.0 | 221.13 ± 2.36 | 0.76 | 0.11 ± 0.01 | 1. 60 | 1.35 ± 0.62 | 1.21 |
| 2.5 | (9.11 ± 0.07) ·10³ | 0.47 | 32.75 ± 7.15 | 0.82 | Nicht bestimmbar nb | nb |
| 5.0 | (93.46 ± 4.88) -10³ | 0.34 | (12.97 ± 0.06) 10³ | 0.53 | nb | nb |

Das rheologische Verhalten der wasserlöslichen Fraktion (1) zeigt sich als abhängig von der Konzentration. Bei verdünnten Dispersionen bis 1 g Präparat pro 100 g Lösung zeigt sich ein viskoses Flüssigkeitsverhalten mit (G "> G"). Das rheologische Verhalten verschob sich zu einem schwachen Gel und dann zu einem Gel, wenn der Gehalt von 1 auf 2,5 und dann auf 10 g / 100 g erhöht wurde. Ein ähnliches Profil wurde auch für Johannisbrotkernmehllösung bei 1 g / 100 g beobachtet, obwohl sein Modul (sowohl G "als auch G") zwischen dem der wasserlöslichen Fraktion (1) bei 2,5 und 5 g / 100 g lag.

Gelartiges Verhalten wurde auch für die Fraktion (3) bei einem Gehalt von 5 und 10 g/100 g beobachtet. Die Dispersionen der unlöslichen Fraktion (6) zeigten über den gesamten Konzentrationsbereich (im Bereich von 1 bis 10 g / 100 g) ein gelartiges Verhalten. In Summe bildeten alle getesteten Fraktionen schwache Gele, die Werte von tan (δ) lagen höher als 0,1.

Der Einfluss der Temperatur auf die rheologischen Eigenschaften der Dispersionen der erfindungsgemäßen Fraktionen in Wasser wurde ebenfalls bewertet. Die wasserlösliche Fraktion (1) erwies sich als temperaturempfindlicher, wobei ein Übergang von Gel zu viskoser Lösung für 5 und 10 g / 100 g beobachtet wurde. Die Fraktion (3) und die unlösliche Fraktion (6) zeigten ebenfalls eine Verringerung sowohl von G "als auch von G", aber beide behielten das Gelverhalten über den getesteten Temperaturbereich (5 bis 80 ° C) bei.

### Ausführungsbeispiel 4

Die Filmbildungseigenschaft der mit Wasser aus dem Ballaststoffpräparat extrahierten wasserlöslichen Fraktion (1) wurde qualitativ bewertet, um zu ermitteln, ob eine essbare Beschichtung oder Süßwaren wie Gummibärchen aus der Fraktion hergestellt werden können. Dazu wurden 20 g einer 2,5%-igen wässrigen Lösung der wasserlöslichen Fraktion in eine Petrischale (Durchmesser 9 cm) gegossen. Die Lösung wurde dann über Nacht bei 25 ° C in einem Ofen mit Luftzirkulation getrocknet. Der erhaltene Film hatte eine bräunliche Farbe, war leicht von der Petrischale ablösbar und formbar. Der wasserlösliche Film zeigte gute Eigenschaften als essbare Beschichtung oder als Basis für Süßwaren, insbesondere, weil es für eine flexible Deformation nicht erforderlich war, Weichmacher zu verwenden.

### Ausführungsbeispiel 5

Ein Pulpenkonzentrat aus Macaubapulpe von A. totai wurde wie in Ausführungsbeispiel 2 beschrieben hergestellt. Das erhaltene Präparat wurde teilweise als Ersatz für Vollei in Muffins eingesetzt wie in Tabelle 6 beschrieben.

**Tabelle 6: Rezeptur von Muffins, hergestellt mit einem erfindungsgemäßen Macaubapulpenkonzentrat**

| **Zutat** | **Anteile der Zutaten (%)** | |
|---|---|---|
| | **Kontrolle nur mit Vollei** | **mit Macauba-BallaststoffKonzentrat** |
| Weizenmehl | 25.0 | 25.0 |
| Zucker | 25.0 | 25.0 |
| Volleipulver | 4.4 | 1.1 |
| Ballaststoffkonzentrat aus (A. totai) | 0.0 | 1.1 |
| Vollmilch | 19.3 | 19.3 |
| Pflanzenöl | 12.5 | 12.5 |
| Backpulver | 1.0 | 1.0 |
| Wasser | 12.9 | 15.1 |
| Summe | 100.0 | 100.0 |

Als Emulgator eignete sich Macauba-Ballaststoffkonzentrat sehr gut für den partiellen Ersatz von Vollei in Muffins. Die erhaltenen Muffins waren der Kontrolle in Aussehen, Textur, Farbe und Geschmack sehr ähnlich.

### Ausführungsbeispiel 6

Ein Pulpenkonzentrat aus Macaubapulpe von A. totai wurde wie in Ausführungsbeispiel 2 beschrieben hergestellt. Die erhaltene Zubereitung wurde zur Herstellung einer Pflanzenmilch wie in Tabelle 7 dargestellt verwendet.

**Tabelle 7: Rezept für Milch auf pflanzlicher Basis, hergestellt mit einem Macaubapulpenkonzentrat gemäß der Erfindung**

| **Zutat** | **Anteile der Zutaten (%)** |
|---|---|
| Wasser | 96.0 |
| Pulpenkonzentrat aus (A. totai) | 2.0 |
| Rapsöl | 1.0 |
| Zucker | 1.0 |
| Summe | 100.0 |

Die Milchzubereitung bestand aus dem Suspendieren von Macaubapulpenkonzentrat in Wasser bei 50°C, bis eine homogene Dispersion erreicht wurde, gefolgt von der Zugabe von Zucker. Danach wurde Rapsöl zugegeben und 5 Minuten bei 21000 U/min emulgiert. Die Mischung wurde mit einem Sieb mit einem Öffnungsdurchmesser von 125 µm filtriert und bei 250 bar in der ersten Stufe und 50 bar in der zweiten Stufe homogenisiert. In der Folge wurde die homogenisierte Milch bei 80 °C für 10 Minuten pasteurisiert und bei 4 °C gelagert. Die resultierende Milch auf Pflanzenbasis wies einen angenehmen Geschmack, ein angenehmes Mundgefühl und eine gute Stabilität auf. Daher ist Macaubapulpenkonzentrat sehr gut für die Herstellung von Milch auf pflanzlicher Basis geeignet.

### Ausführungsbeispiel 7

Ein Pulpenkonzentrat aus Macaubapulpe von A. totai wurde wie in Ausführungsbeispiel 2 beschrieben hergestellt. Das erhaltene Präparat wurde verwendet, um eine zweite Art pflanzlicher Milch herzustellen, wie in Tabelle 8 dargestellt.

**Tabelle 8: Rezept für Milch auf pflanzlicher Basis (2), hergestellt mit einem Macaubapulpenkonzentrat gemäß der Erfindung**

| **Zutat** | **Anteile der Zutaten (%)** |
|---|---|
| Wasser | 95.97 |
| Ballaststoffkonzentrat aus (A. totai) | 2.00 |
| Rapsöl | 1.00 |
| Zucker | 1.00 |
| Enzympräparat | 0.03 |
| Summe | 100.00 |

Die Milchzubereitung bestand aus dem Suspendieren von Macaubapulpenkonzentrat in Wasser bei 50°C, bis die Dispersion homogen aussah, gefolgt von der Zugabe von Zucker. Anschließend wurde eine Enzymzubereitung bestehend aus Hemicellulasen und Pektinasen zugegeben und die Mischung 1 Stunde bei 50°C inkubiert. Anschließend wurde Rapsöl zugegeben und 5 Minuten bei 21000 U/min emulgiert. Die Mischung wurde mit einem Sieb mit einem Öffnungsdurchmesser von 125 µm filtriert und bei 250 bar in der ersten Stufe und 50 bar in der zweiten Stufe homogenisiert. In der Folge wurde die homogenisierte Milch bei 80 °C für 10 Minuten pasteurisiert und bei 4 °C gelagert.

Die resultierende Milch auf Pflanzenbasis wies einen angenehmen Geschmack, ein angenehmes Mundgefühl und eine angenehme Stabilität auf. Diese Pflanzenmilch hatte einen neutraleren Geschmack und eine niedrigere Viskosität als die Pflanzenmilch aus Beispiel 6.

Quellen:
1. Mudgil, D. and S. Barak, Composition, properties and health benefits of indigestible carbohydrate polymers as dietary fiber: a review. International journal of biological macromolecules, 2013. 61: p. 1-6.
2. Padayachee, A., et al., Complexity and health functionality of plant cell wall fibers from fruits and vegetables. Critical reviews in food science and nutrition, 2017. 57(1): p. 59-81.
3. Cui, S.W. and Q. Wang, Cell wall polysaccharides in cereals: chemical structures and functional properties. Structural Chemistry, 2009. 20(2): p. 291-297.
4. Lescano, C., et al., Nutrients content, characterization and oil extraction from Acrocomia aculeata (Jacq.) Lodd. fruits. African Journal of Food Science, 2015. 9(3): p. 113-119.
5. Trentini CP, Oliveira DM, Zanette CM, Silva C. Lowpressure solvent extraction of oil from Macauba (Acrocomia aculeata) pulp: characterization of oil and defatted meal. Ciência Rural, Santa Maria. 2016; 46(4): 725-731.
6. Silva AO, Cortez-Verga WR, Prentice C, Fonseca GG. Development and characterization of biopolymer films based on bocaiuva (Acrocomia aculeata) flour. International Journal of Biological Macromolecules. 2020; 155: 1157-1168.
7. Colombo CA, Berton LHC, Diaz BG, Ferrari RA. Macauba: a promising tropical palm for the production of vegetable oil. OCL. 2018; 25(1): D108.
8. AOAC International. (2000). Method 991.43 Total dietary fiber. Enzymatic-gravimetric method. In Official methods of analysis of the association of official analytical chemists (edition 17th). Gaitherburg, MD, USA: Association of Official Analytical Chemists.
9. AOAC International. Method 968.06 Protein (crude) in animal feed. Dumas Method. In Official Methods of Analysis of the Association of Official Analytical Chemists, edition 15th; Association of Official Analytical Chemists: Arlington, VA, USA, 1990.
10. AOAC International. Method 979.09 Protein in grains. Official methods of analysis, 16th ed. Washington DC, USA: Association of Official Analytical Chemists, 1995. 109 p.
11. Pearce, K.N., Kinsella, J.E. Emulsifying properties of proteins: evaluation of a turbudimetric technique. Journal of Agricultural and Food Chemistry, v. 26, p. 716-723, 1978.
12. Wasche, A., Muller, K., Knauf, U., "New processing of lupin protein isolates and functional properties". Nahrung/Food, 2001, 45, 393-395
13. AOAC International. Method 920.39 Fat (crude) or Ether Extract. In Official Methods of Analysis of the Association of Official Analytical Chemists, edition 15th; Association of Official Analytical Chemists: Arlington, VA, USA, 1990.
14. German Food Act. (1980). Methods L.01.00-60, L. 16.01-2, L. 17.00-1, L. 17.00-3, 1980. In BVL Bundesamt fuer Verbraucherschutz und Lebensmittelsicherheit. Berlin, Germany: Beuth Verlag GmbH. Amtliche Sammlung von Untersuchungsverfahren nach Â§ 64 LFGB, Â§ 35 Vorlaeufiges Tabakgesetz, Â§ 28b GenTG-I-Lebensmittel-Band I (L) Verfahren zur Probenahme und Untersuchung von Lebensmitteln.
15. AACC. Method 56-30. Approved methods of the aacc. 10th ed.; American Association of Cereal Chemists: St. Paul, MN, USA, 2000.
16. Muranyi IS, Otto C, Pickardt C, Osen R, Koehler P, Schweiggert-Weisz U. Influence of isolation method on the technofunctional properties of protein isolates from Lupinus angustifolius L. Journal of Food Science. 2016; 81(11): C2656-C2663.

## Patentansprüche

1. Ballaststoffpräparat, welches aus Fruchtfleisch von Macaubafrüchten hergestellt ist und
- einen Ballaststoffgehalt größer 25 Mass.-%, bevorzugt größer 30 Mass.-%, bestimmt nach der Referenzmethode 991.43 von AOAC International,
- einen Fettgehalt kleiner 20 Mass.-%, bevorzugt kleiner 10 Mass.-%,
- einen Wassergehalt kleiner 20 Mass.-%, bevorzugt kleiner 15 Mass.-%, und
- eine helle Farbe mit einem L*-Wert, bestimmt gemäß CIE-L*a*b*-Farbmessung, größer 84 aufweist,
- wobei ein Anteil an alkohol-wasserlöslichen Substanzen am Ballaststoffpräparat bezogen auf die Masse des Präparates weniger als 40% beträgt.

2. Ballaststoffpräparat nach Anspruch 1, **dadurch gekennzeichnet,**
**dass** der Anteil an alkohol-wasserlöslichen Substanzen geringer als 35 Mass.-%, vorteilhaft kleiner 30 Mass.-% ist.

3. Ballaststoffpräparat nach Anspruch 1 oder 2,
**dadurch gekennzeichnet,**
**dass** es einen Fettgehalt kleiner 5 Mass.-%, vorteilhaft kleiner 3 Mass.-%, besonders vorteilhaft kleiner 2 Mass.-% aufweist.

4. Ballaststoffpräparat nach einem der Ansprüche 1 bis 3, **dadurch gekennzeichnet,**
**dass** es einen Ballaststoffgehalt größer 40 Mass.-%, bevorzugt größer 50 Mass.-%, besonders vorteilhaft größer 60 Mass.-% aufweist.

5. Ballaststoffpräparat nach einem der Ansprüche 1 bis 4, **dadurch gekennzeichnet,**
**dass** es eine helle Farbe mit einem L*-Wert, bestimmt gemäß CIE-L*a*b*-Farbmessung, größer 90, vorteilhaft größer 95 aufweist.

6. Ballaststoffpräparat nach einem der Ansprüche 1 bis 5, **dadurch gekennzeichnet,**
**dass** es eine oder mehrere der folgenden Eigenschaften aufweist:
- einen Emulgieraktivitätsindex von größer 35 m²/g trocknem Präparat, bevorzugt größer 55 m²/g trocknem Präparat, besonders bevorzugt größer 70 m²/g trocknem Präparat, und/oder eine Emulsionsstabilität von größer 30 Minuten, bevorzugt größer 60 Minuten, besonders bevorzugt größer 120 Minuten,
- eine Emulgierkapazität von größer 400 mL/g trocknem Präparat auf, besser größer 500 mL/g trocknem Präparat, besonders größer 600 mL/g trocknem Präparat,
- eine Schaumaktivität größer 200 Vol.-%, bevorzugt größer 400 Vol.-%, besonders bevorzugt größer 600 Vol.-%, und eine Schaumstabilität von größer 25 Vol.-%, bevorzugt größer 50 Vol.-%, besonders bevorzugt größer 75 Vol.-%,
- eine Wasserbindekapazität von größer als 1,5 mL/g TS, bevorzugt größer 3 mL/g TS, besonders bevorzugt größer 4 mL/g TS, und
- eine Ölbindekapazität von größer als 1,5 mL/g TS, bevorzugt größer 3,5 mL/g TS, besonders bevorzugt größer 5 mL/g TS.

7. Verfahren zur Herstellung eines Ballaststoffpräparates aus Fruchtfleisch von Macaubafrüchten mit wenigstens folgenden Schritten:
- Bereitstellen von teilentölter Pulpe aus Macaubafrüchten mit einem Fettgehalt zwischen 3 Mass.-% und 60 Mass.-% bezogen auf Trockenmasse;
- Reduktion des Fettgehaltes in der teilentölten Pulpe mit Hilfe eines oder mehrerer extraktiver Verfahren auf einen Wert kleiner 20 Mass.-%, vorzugsweise kleiner 10 Mass.-%, falls der Fettgehalt der teilentölten Pulpe größer ist, und
- Reduktion eines Gehaltes an alkohol-wasserlöslichen Substanzen in der Pulpe mit Hilfe eines oder mehrerer extraktiver Verfahren oder einer durch wässrige Extraktion aus der Pulpe erhaltenen Fraktion durch Fällung oder durch Trockenfraktionierung auf Werte kleiner 40 Mass.-%, bevorzugt kleiner 35 Mass.-%, bezogen auf Trockenmasse.

8. Verfahren nach Anspruch 7,
**dadurch gekennzeichnet,**
**dass** die teilentölte Pulpe mit einem Schalengehalt von weniger als 10 %, besser weniger als 5 %, vorzugsweise weniger als 2 Massen-%, bezogen auf die Trockenmasse und einem Endokarp- und Kerngehalt von weniger als 3 %, besser weniger als 2 %, vorzugsweise weniger als 1 Massen-% bezogen auf die Trockenmasse bereitgestellt wird.

9. Verfahren nach Anspruch 7 oder 8,
**dadurch gekennzeichnet,**
**dass** der Fettgehalt in der Pulpe auf einen Wert kleiner 5 Mass.%, vorzugsweise kleiner 3 Mass.-%, bevorzugt kleiner 2 Mass.-% reduziert wird.

10. Verfahren nach einem der Ansprüche 7 bis 9,
**dadurch gekennzeichnet,**
**dass** ein Anteil an Fett und alkohol-wasserlöslichen Substanzen mit einer oder mehreren Mischungen aus Ethanol und Wasser im Massenverhältnis von 94:6 bis 90:10 bei Temperaturen von 40-70°C, vorteilhaft 50-65°C, simultan aus der Pulpe abgetrennt werden.

11. Verfahren nach Anspruch 10,
**dadurch gekennzeichnet,**
**dass** eine weitere Reduktion des Anteiles an Fett und alkohol-wasserlöslichen Substanzen in der Pulpe durch Nutzung von Wasser und/oder einer oder mehreren Mischungen aus Alkohol und Wasser im Massenverhältnis kleiner 90:10, besser kleiner 80:20, und bei einer Temperatur erfolgt, die vorzugsweise zwischen 40 und 90 °C liegt.

12. Verfahren nach einem der Ansprüche 7 bis 11,
**dadurch gekennzeichnet,**
**dass** dem Ballaststoffpräparat Macaubaproteinmehl oder andere Pflanzenproteinpräparate in einer Menge zugegeben werden, dass das Ballaststoffpräparat nach der Zugabe einen Proteinanteil von 10 bis 90 Mass.-%, vorteilhaft 20 bis 80 Mass.-%, besonders vorteilhaft 40 bis 60 Mass.-% aufweist.

13. Verfahren nach einem der Ansprüche 7 bis 12,
**dadurch gekennzeichnet,**
**dass** durch das eine oder die mehreren extraktiven Verfahren zur Reduktion des Gehaltes an alkohol-wasserlöslichen Substanzen aus der Pulpe ein Konzentrat erhalten wird, das einer wässrigen Extraktion und einer anschließenden Fällung mit Alkohol und Trocknung unterzogen wird, um ein wasserlösliches Ballaststoffpräparat zu erhalten.

14. Verfahren nach einem der Ansprüche 7 bis 12,
**dadurch gekennzeichnet,**
**dass** die durch wässrige Extraktion aus der Pulpe erhaltene und anschließend einer Fällung unterzogene Fraktion getrocknet wird, um ein wasserlösliches Ballaststoffpräparat zu erhalten.

15. Verfahren nach Anspruch 13 oder 14,
**dadurch gekennzeichnet,**
**dass** eine bei der wässrigen Extraktion verbleibende zweite Fraktion wasserunlöslicher Bestandteile als wasserunlösliches Ballaststoffpräparat bereitgestellt wird.

16. Verfahren nach Anspruch 13 oder 14,
**dadurch gekennzeichnet,**
**dass** eine bei der wässrigen Extraktion verbleibende zweite Fraktion wasserunlöslicher Bestandteile mittels NaOH-EDTA-Lösung mit 0,05-0,1 mol/L NaOH oder Natriumcarbonat und 0,5 mmol EDTA oder CDTA oder 0,5% (m/v) Ammoniumoxalatlösung in eine lösliche dritte Fraktion und eine unlösliche vierte Fraktion aufgetrennt und die dritte Fraktion anschließend getrocknet oder einer alkoholischen Fällung unterzogen wird.

17. Verfahren nach Anspruch 16,
**dadurch gekennzeichnet,**
**dass** die vierte Fraktion mittels konzentrierter Lauge in eine in der Lauge lösliche fünfte Fraktion und eine in der Lauge unlösliche sechste Fraktion aufgetrennt und die fünfte Fraktion neutralisiert und getrocknet oder einer alkoholischen Fällung unterzogen wird.

18. Wasserlösliches Ballaststoffpräparat, welches aus Fruchtfleisch von Macaubafrüchten hergestellt ist und
- einen Ballaststoffgehalt größer 60 Mass.-%, bevorzugt größer 70 Mass.-%, besser mehr als 80 Mass.-%, bestimmt nach der Referenzmethode 991.43 von AOAC International, und
- einen Fettgehalt kleiner 20 Mass.-%, bevorzugt kleiner 10 Mass.-%, besonders bevorzugt weniger als 5 Mass.-% aufweist,
- wobei ein Anteil an alkohol-wasserlöslichen Substanzen am Ballaststoffpräparat bezogen auf die Masse des Präparates weniger als 20 %, vorzugsweise weniger als 15 %, besser weniger als 10 Masse-% beträgt.

19. Wasserlösliches Ballaststoffpräparat nach Anspruch 18, **dadurch gekennzeichnet,**
**dass** es eine oder mehrere der folgenden Eigenschaften aufweist, besser mehrere:
- eine Viskosität einer 1% Dispersion in Wasser (m/v) bei einer Schergeschwindigkeit von 100 s-1 und 25°C höher als 50 cP, besser höher als 70 cP, besser höher als 80 cP;
- eine Viskosität einer 5% Dispersion in Wasser (m/v) bei einer Schergeschwindigkeit von 100 s-1 und 25°C höher als 3000 cP, vorzugsweise höher als 4000 cP, besser höher als 6000 cP;
- ein Ölbindevermögen von größer 0,5 ml/g TS, vorzugsweise größer 1,0 ml/g TS.

20. Wasserunlösliches Ballaststoffpräparat, welches aus Fruchtfleisch von Macaubafrüchten hergestellt ist und
- einen Ballaststoffgehalt größer 60 Mass.-%, bevorzugt größer 70 Mass.-%, besser mehr als 80 Mass.-%, bestimmt nach der Referenzmethode 991.43 von AOAC International, und
- einen Fettgehalt kleiner 20 Mass.-%, bevorzugt kleiner 10 Mass.-%, besonders bevorzugt weniger als 5 Mass.-% aufweist,
- wobei ein Anteil an alkohol-wasserlöslichen Substanzen am Ballaststoffpräparat bezogen auf die Masse des Präparates weniger als 20 %, vorzugsweise weniger als 15 %, besser weniger als 10 Masse-% beträgt.

21. Wasserunlösliches Ballaststoffpräparat nach Anspruch 20, **dadurch gekennzeichnet,**
**dass** es eine oder mehrere der folgenden Eigenschaften aufweist, besser mehrere:
- ein Wasserbindevermögen von größer 1,0 ml/g TS, bevorzugt größer 3 ml/g TS, besonders bevorzugt größer 4 ml/g TS, noch besser größer 6 ml/g TS;
- ein Ölbindevermögen von größer 1,0 ml/g TS, bevorzugt größer 3,0 ml/g TS, besonders bevorzugt größer 4 ml/g TS, noch besser größer 6 ml/g TS.

## Claims

1. Dietary fibre preparation that is produced from the flesh of macauba fruit and has
- a dietary fibre content of more than 25% by mass, preferably more than 30% by mass, determined according to the AOAC International reference method 991.43,
- a fat content lower than 20% by mass, preferably lower than 10% by mass,
- a water content lower than 20% by mass, preferably lower than 15% by mass, and
- a bright colour with a L* value, determined according to CIE-L*a*b* colorimetry, greater than 84,
- wherein a content of alcohol-water soluble substances in the dietary fibre preparation constitutes less than 40% relative to the mass of the preparation.

2. Dietary fibre preparation according to Claim 1, **characterized in that**
the content of alcohol-water soluble substances is less than 35% by mass, advantageously less than 30% by mass.

3. Dietary fibre preparation according to Claim 1 or 2, **characterized in that**
it has a fat content lower than 5% by mass, advantageously lower than 3% by mass, particularly advantageously lower than 2% by mass.

4. Dietary fibre preparation according to any one of Claims 1 to 3,
**characterized in that**
it has a dietary fibre content of more than 40% by mass, preferably more than 50% by mass, particularly advantageously more than 60% by mass.

5. Dietary fibre preparation according to any one of Claims 1 to 4,
**characterized in that**
it has a bright colour with a L*-value, determined according to CIE-L*a*b* colorimetry, higher than 90, advantageously higher than 95.

6. Dietary fibre preparation according to any one of Claims 1 to 5,
**characterized in that**
it has one or more of the following properties:
- an emulsifying activity index of more than 35 m²/g dry preparation, preferably more than 55 m²/g dry preparation, particularly preferably more than 70 m²/g dry preparation, and/or an emulsion stability longer than 30 minutes, preferably longer than 60 minutes, particularly preferably more than 120 minutes,
- an emulsifying capacity of more than 400 mL/g dry preparation, better more than 500 mL/g dry preparation, particularly more than 600 mL/g dry preparation,
- a foaming activity of more than 200% by volume, preferably more than 400% by volume, particularly preferably more than 600% by volume, and a foam stability of more than 25% by volume, preferably more than 50% by volume, particularly preferably more than 75% by volume,
- a water binding capacity of more than 1.5 mL/g DS, preferably more than 3 mL/g DS, particularly preferably more than 4 mL/g DS, and
- an oil binding capacity of more than 1.5 mL/g DS, preferably more than 3.5 mL/g DS, particularly preferably more than 5 mL/g DS.

7. Method for producing a dietary fibre preparation from the flesh of macauba fruit, including at least the following steps:
- Providing partially de-oiled pulp from macauba fruits, with a fat content between 3% by mass and 60% by mass relative to dry substance;
- Reducing the fat content in the partially de-oiled pulp with the aid of one or more extraction methods to a value less than 20% by mass, preferably less than 10% by mass if the fat content of the partially de-oiled pulp is greater, and
- Reducing a content of alcohol-water soluble substances in the pulp with the aid of one or more extraction methods or in a fraction obtained from the pulp by aqueous extraction through precipitation or dry fractionation to values less than 40% by mass, preferably less than 35% by mass, relative to dry substance.

8. Method according to Claim 7,
**characterized in that**
the partially de-oiled pulp is provided with a shell content of less than 10%, better less than 5%, preferably less than 2% by mass, relative to dry substance, and an endocarp and kernel content of less than 3%, better less than 2%, preferably less than 1% by mass relative to the dry substance.

9. Method according to Claim 7 or 8,
**characterized in that**
the fat content in the pulp is reduced to a value less than 5% by mass, preferably less than 3% by mass, more preferably less than 2% by mass.

10. Method according to any one of Claims 7 to 9,
**characterized in that**
a content of fat and alcohol-water soluble substances is separated out of the pulp simultaneously with one or more mixtures of ethanol and water in a mass ratio from 94:6 to 90:10 at temperatures of 40-70°C, advantageously 50-65°C.

11. Method according to Claim 10,
**characterized in that**
a further reduction in the content of fat and alcohol-water soluble substances in the pulp is carried out by the use of water and/or one or more mixtures of alcohol and water in a mass ratio less than 90:10, better less than 80:20, and at a temperature preferably between 40 and 90°C.

12. Method according to any one of Claims 7 to 11, **characterized in that**
macauba protein flour or other plant protein preparations are added to the dietary fibre preparation in such a quantity that after the addition the dietary fibre preparation has a protein content of 10 to 90% by mass, advantageously 20 to 80% by mass, particularly advantageously 40 to 60%.

13. Method according to any one of Claims 7 to 12, **characterized in that**
through the one or more extraction methods for reducing the content of alcohol-water soluble substances a concentrate is obtained from the pulp, and undergoes an aqueous extraction and subsequent precipitation with alcohol and drying to obtain a water-soluble dietary fibre preparation.

14. Method according to any one of Claims 7 to 12, **characterized in that**
the fraction that is obtained from the pulp by aqueous extraction and subsequently undergoes a precipitation step is dried to obtain a water-soluble dietary fibre preparation.

15. Method according to Claim 13 or 14,
**characterized in that**
a second fraction of water insoluble components left by the aqueous extraction is provided as a water insoluble dietary fibre preparation.

16. Method according to Claim 13 or 14,
**characterized in that**
a second fraction of water insoluble components left by the aqueous extraction is separated into a soluble third fraction and an insoluble fourth fraction by means of NaOH-EDTA solution with 0.05-0.1 mol/L NaOH or sodium carbonate and 0.5 mmol EDTA or CDTA or 0.5% (m/v) ammoniumoxalate solution, and the third fraction is subsequently dried or undergoes an alcohol precipitation.

17. Method according to Claim 16,
**characterized in that**
the fourth fraction is separated by means of concentrated alkaline solution into a fifth fraction which is soluble in the concentrated alkaline solution and a sixth fraction which is insoluble in the concentrated alkaline solution, and the fifth fraction is neutralised and dried or undergoes an alcohol precipitation.

18. Water soluble dietary fibre preparation, which is produced from flesh of the macauba fruit and has
- a dietary fibre content of more than 60% by mass, preferably more than 70% by mass, better more than 80% by mass, determined according to the AOAC International reference method 991.43, and
- a fat content lower than 20% by mass, preferably lower than 10% by mass, particularly preferably lower than 5% by mass,
- wherein a content of alcohol-water soluble substances in the dietary fibre preparation is less than 20%, preferably less than 15%, better less than 10% by mass relative to the mass of the preparation.

19. Water soluble dietary fibre preparation according to Claim 18,
**characterized in that**
it has one or more of the following properties, better several:
- a viscosity of a 1% dispersion in water (m/v) with a shear rate of 100 s-1 and 25°C higher than 50 cP, better higher than 70 cP, better higher than 80 cP;
- a viscosity of a 5% dispersion in water (m/v) with a shear rate of 100 s-1 and 25°C higher higher than 3000 cP, preferably higher than 4000 cP, better higher than 6000 cP;
- an oil binding capacity greater than 0.5 ml/g DS, preferably greater than 1.0 ml/g DS.

20. Water insoluble dietary fibre preparation, which is produced from flesh of the macauba fruit and has
- a dietary fibre content of more than 60% by mass, preferably more than 70% by mass, better more than 80% by mass, determined according to the AOAC International reference method 991.43, and
- a fat content lower than 20% by mass, preferably lower than 10% by mass, particularly preferably lower than 5% by mass,
- wherein a content of alcohol-water soluble substances in the dietary fibre preparation is less than 20%, preferably less than 15%, better less than 10% by mass relative to the mass of the preparation.

21. Water insoluble dietary fibre preparation according to Claim 20,
**characterized in that**
it has one or more of the following properties, better several:
- a water binding capacity greater than 1.0 ml/g DS, preferably greater than 3 ml/g DS, particularly preferably greater than 4 ml/g DS, better still greater than 6 ml/g DS;
- an oil binding capacity greater than 1.0 ml/g DS, preferably greater than 3.0 ml/g DS, particularly preferably greater than 4 ml/g DS, still better greater than 6 ml/g DS.

## Revendications

1. Préparation contenant des fibres, laquelle est produite à partir de pulpe de fruits du macauba et
- fait preuve d'une teneur en fibres supérieure à 25 % en masse, de manière préférentielle supérieure à 30 % en masse, déterminée selon la méthode de référence 991.43 de l'AOAC International,
- fait preuve d'une teneur en matières grasses inférieure à 20 % en masse, de manière préférentielle inférieure à 10 % en masse,
- fait preuve d'une teneur en eau inférieure à 20 % en masse, de manière préférentielle inférieure à 15 % en masse et
- présente une couleur claire, avec une valeur L*, déterminée selon la mesure de la couleur CIE L*a*b* supérieure à 84,
- une proportion de substances solubles dans l'alcool et dans l'eau de la préparation contenant des fibres, rapportée à la masse de la préparation s'élevant à moins de 40 %.

2. Préparation contenant des fibres selon la revendication 1,
**caractérisée**
**en ce que** la proportion de substances solubles dans l'alcool et dans l'eau est plus faible que 35 % en masse, avantageusement inférieure à 30 % en masse.

3. Préparation contenant des fibres selon la revendication 1 ou 2,
**caractérisée**
**en ce qu'**elle fait preuve d'une teneur en matières grasses inférieure à 5 % en masse, avantageusement inférieure à 3 % en masse, de manière particulièrement avantageuse, inférieure à 2 % en masse.

4. Préparation contenant des fibres selon l'une quelconque des revendications 1 à 3,
**caractérisée**
**en ce qu'**elle fait preuve d'une teneur en fibres supérieure à 40 % en masse, de manière préférentielle supérieure à 50 % en masse, de manière particulièrement avantageuse, supérieure à 60 % en masse.

5. Préparation contenant des fibres selon l'une quelconque des revendications 1 à 4,
**caractérisée**
**en ce qu'**elle présente une couleur claire, d'une valeur L*, déterminée selon la mesure de la couleur CIE L*a*b*, supérieure à 90, avantageusement supérieure à 95.

6. Préparation contenant des fibres selon l'une quelconque des revendications 1 à 5,
**caractérisée**
**en ce qu'**elle fait preuve d'une ou de plusieurs des propriétés suivantes :
- un indice d'activité émulsifiante supérieur à 35 m²/g de préparation sèche, de manière préférentielle supérieur à 55 m²/g de préparation sèche, de manière particulièrement préférentielle, supérieur à 70 m²/g de préparation sèche, et / ou une stabilité de l'émulsion supérieure à 30 minutes, de manière préférentielle supérieure à 60 minutes, de manière particulièrement préférentielle, supérieure à 120 minutes,
- une capacité d'émulsion supérieure à 400 mL/g de préparation sèche, préférablement supérieure à 500 mL/g de préparation sèche, particulièrement supérieure à 600 mL/g de préparation sèche,
- une activité moussante supérieure à 200 % en volume, de manière préférentielle supérieure à 400 % en volume, de manière particulièrement préférentielle, supérieure à 600 % en volume et une stabilité de la mousse supérieure à 25 % en volume, de manière préférentielle supérieure à 50 % en volume, de manière particulièrement préférentielle, supérieure à 75 % en volume,
- une capacité d'absorption d'eau supérieure à 1,5 mL/g TS, de manière préférentielle supérieure à 3 mL/g TS, de manière particulièrement préférentielle, supérieure à 4 mL/g TS et
- une capacité d'absorption d'huile supérieure à 1,5 mL/g TS, de manière préférentielle supérieure à 3,5 mL/g TS, de manière particulièrement préférentielle, supérieure à 5 mL/g TS.

7. Procédé, destiné à produire une préparation contenant des fibres à partir de pulpe de fruits du macauba, comportant au moins les étapes suivantes, consistant à :
- mettre à disposition une pulpe de fruits du macauba partiellement déshuilée, faisant preuve d'une teneur en matières grasses comprise entre 3 % en masse et 60 % en masse, rapportée à la masse sèche ;
- à l'aide d'un ou de plusieurs procédés d'extraction, réduire la teneur en matières grasses dans la pulpe partiellement déshuilée à une valeur inférieure à 20 % en masse, de préférence inférieure à 10 % en masse, si la teneur en matières grasses de la pulpe partiellement déshuilée est supérieure et
- à l'aide d'un ou de plusieurs procédés d'extraction ou d'une fraction obtenue par extraction aqueuse hors de la pulpe par précipitation ou par fractionnement à sec, réduire une teneur en substances solubles dans l'alcool et dans l'eau dans la pulpe à des valeurs inférieures à 40 % en masse, de manière préférentielle inférieures à 35 % en masse, rapportée à la masse sèche.

8. Procédé selon la revendication 7,
**caractérisé**
**en ce que** l'on met à disposition la pulpe partiellement déshuilée avec une teneur en coques de moins de 10 %, préférablement de moins de 5 %, de préférence de moins de 2 % en masse, rapportée à la masse sèche et avec une teneur en endocarpe et en pépins de moins de 3 %, préférablement de moins de 2 %, de préférence de moins de 1 % en masse, rapportée à la masse sèche.

9. Procédé selon la revendication 7 ou 8,
**caractérisé**
**en ce que** l'on réduit la teneur en matières grasses dans la pulpe à une valeur inférieure à 5 % en masse, de préférence inférieure à 3 % en masse, de manière préférentielle, inférieure à 2 % en masse.

10. Procédé selon l'une quelconque des revendications 7 à 9,
**caractérisé**
**en ce que** l'on sépare simultanément de la pulpe une proportion de matières grasses et de substances solubles à l'alcool et à l'eau avec un ou plusieurs mélanges d'éthanol et d'eau dans un rapport massique de 94:6 à 90:10 à des températures de 40 à 70 °C, avantageusement de 50 à 65 °C.

11. Procédé selon la revendication 10,
**caractérisée**
**en ce que** l'on procède à une réduction supplémentaire de la proportion de matières grasses et de substances solubles dans l'alcool et dans l'eau dans la pulpe en utilisant de l'eau et/ou un ou plusieurs mélanges d'alcool et d'eau dans un rapport massique inférieur à 90:10, préférablement inférieur à 80:20 et à une température qui se situe e préférence entre 40 et 90 °C.

12. Procédé selon l'une quelconque des revendications 7 à 11,
**caractérisé**
**en ce que** l'on rajoute à la préparation contenant des fibres de la farine protéinique du fruit du macauba ou d'autres préparations protéiniques végétales dans une quantité telle qu'après l'ajout, la préparation contenant des fibres fasse preuve d'une teneur en protéines de 10 à 90 % en masse, avantageusement de 20 à 80 % en masse, de manière particulièrement avantageuse, de 40 à 60 % en masse.

13. Procédé selon l'une quelconque des revendications 7 à 12,
**caractérisé**
**en ce que** par l'un ou par les plusieurs procédés d'extraction destiné(s) à réduire la teneur en substances solubles dans l'alcool et dans l'eau dans la pulpe, l'on obtient un concentré que l'on soumet à une précipitation ultérieure avec de l'alcool et à un séchage, pour obtenir une préparation contenant des fibres.

14. Procédé selon l'une quelconque des revendications 7 à 12,
**caractérisé**
**en ce que** l'on fait sécher la fraction obtenue par extraction aqueuse hors de la pulpe et soumise ensuite à une précipitation, pour obtenir une préparation contenant des fibres soluble dans l'eau.

15. Procédé selon la revendication 13 ou 14,
**caractérisé**
**en ce que** l'on met à disposition une deuxième fraction de composants non solubles dans l'eau restant lors de l'extraction aqueuse en tant que préparation contenant des fibres non soluble dans l'eau.

16. Procédé selon la revendication 13 ou 14,
**caractérisé**
**en ce que** l'on sépare la deuxième fraction de composants non solubles dans l'eau restant lors de l'extraction aqueuse au moyen d'une solution NaOH-EDTA, à raison de de 0,05 à 0,1 mole/L de NaOH ou de carbonate de sodium et de 0,5 mmole d'EDTA ou de CDTA ou de 0,5% (m/v) de solution d'oxalate d'ammonium en une troisième fraction soluble et en une quatrième fraction insoluble et l'on fait ensuite sécher la troisième fraction ou on la soumet à une précipitation alcoolique.

17. Procédé selon la revendication 16,
**caractérisé**
**en ce que** l'on sépare la quatrième fraction au moyen d'une lessive concentrée en une cinquième fraction soluble dans la lessive et en une sixième fraction, non soluble dans la lessive et on neutralise et on fait sécher la cinquième fraction ou on la soumet à une précipitation alcoolique.

18. Préparation contenant des fibres soluble dans l'eau, laquelle est produite à partie de pulpe de fruits du macauba et
- fait preuve d'une teneur en fibres supérieure à 60 % en masse, de manière préférentielle supérieure à 70 % en masse, préférablement, de plus de 80 % en masse, déterminée selon la méthode de référence 991.43 de l'AOAC International et
- fait preuve d'une teneur en matières grasses inférieure à 20 % en masse, de manière préférentielle inférieure à 10 % en masse, de manière particulièrement préférentielle de moins de 5 % en masse,
- une proportion de substances solubles dans l'alcool et dans l'eau de la préparation contenant des fibres, rapportée à la masse de la préparation s'élevant à moins de 20 %, de préférence à moins de 15 %, préférablement à moins de 10 % en masse.

19. Préparation contenant des fibres soluble dans l'eau selon la revendication 18,
**caractérisée**
**en ce qu'**elle fait preuve d'une ou de plusieurs, préférablement de plusieurs des propriétés suivantes :
- une viscosité d'une dispersion à raison de 1 % dans de l'eau (m/v) à une vitesse de cisaillement de 100 s-1 et à 25 °C plus élevée que 50 cP, préférablement plus élevée que 70 cP, préférablement plus élevée que 80 cP ;
- une viscosité d'une dispersion à raison de 5 % dans de l'eau (m/v) à une vitesse de cisaillement de 100 s-1 et à 25 °C plus élevée que 3000 cP, de préférence plus élevée que 4000 cP, préférablement plus élevée que 6000 cP;
- un pouvoir d'absorption d'huile supérieur à 0,5 ml/g TS, de préférence supérieur à 1,0 ml/g TS.

20. Préparation contenant des fibres insoluble dans l'eau, laquelle est produite à partir de pulpe de fruits du macauba et
- fait preuve d'une teneur en fibres supérieure à 60 % en masse, de manière préférentielle supérieure à 70 % en masse, préférablement, de plus de 80 % en masse, déterminée selon la méthode de référence 991.43 de l'AOAC International et
- fait preuve d'une teneur en matières grasses inférieure à 20 % en masse, de manière préférentielle inférieure à 10 % en masse, de manière particulièrement préférentielle de moins de 5 % en masse,
- une proportion de substances solubles dans l'alcool et dans l'eau dans la préparation contenant des fibres, rapportée à la masse de la préparation s'élevant à moins de 20 %, de préférence à moins de 15 %, préférablement à moins de 10 % en masse.

21. Préparation contenant des fibres non soluble dans l'eau selon la revendication 20,
**caractérisée**
**en ce qu'**elle fait preuve d'une ou de plusieurs, préférablement de plusieurs des propriétés suivantes:
- un pouvoir d'absorption d'eau supérieur à 1,0 ml/g TS, de manière préférentielle supérieur à 3 ml/g TS, de manière particulièrement préférentielle, supérieur à 4 ml/g TS, de manière encore plus préférable, supérieur à 6 ml/g TS;
- un pouvoir d'absorption d'huile supérieur à 1,0 ml/g TS, de manière préférentielle supérieur à 3,0 ml/g TS, de manière particulièrement préférentielle, supérieur à 4 ml/g TS, de manière encore plus préférable, supérieur à 6 ml/g TS.
